# EUROPEAN PATENT SPECIFICATION

(11) **EP 1 549 329 B1**
(45) Date of publication and mention of the grant of the patent: **27.12.2006**
(21) Application number: 03757940.6
(22) Date of filing: 09.10.2003
(51) Int. Cl.: A61K 36/27, A61P 35/00, A61P 39/00

(54) **EXTRACT WITH ANTI-TUMOR AND ANTI-POISONOUS ACTIVITY**
EXTRAKT MIT ANTITUMORALER UND ANTITOXISCHER WIRKUNG
EXTRAIT PRESENTANT UNE ACTIVITE ANTICANCEREUSE ET UNE ACTIVITE ANTITOXIQUE

(30) Priority: 09.10.2002 WO PCT/EP02/11310
(43) Date of publication of application: 06.07.2005
(73) Proprietor: Unibioscreen S.A., 1070 Bruxelles (BE); Université Libre de Bruxelles, 1050 Brussels (BE)
(72) Inventor: DARRO, Francis, 1180 Uccle (BE); BRAEKMAN, Jean-Claude, 1640 Rhode-Saint-Genèse (BE); GUISSOU, Pierre, Ouagadougou (BF); NACOULMA, Odile, Germaine, Ouagadougou (BF); EL YAZIDI, Mohamed, 1083 Ganshoren (BE); DEWELLE, Janique, 6238 Luttre (BE); VAN GINKEL, Rob, 2290 Vorselaar (BE); VAN DAMME, Marc, 1050 Bruxelles (BE); KISS, Robert, 1600 Sint-Pieters-Leeuw (BE)
(74) Representative: Brants, Johan P.E.
(86) International application number: PCT/EP2003/011192
(87) International publication number: WO 2004/032948

(56) References cited:
- SMIT H F ET AL: "AYURVEDIC HERBAL DRUGS WITH POSSIBLE CYTOSTATIC ACTIVITY" JOURNAL OF ETHNOPHARMACOLOGY, ELSEVIER SCIENTIFIC PUBLISHERS LTD, IE, vol. 47, 1995, pages 75-84, XP000885469 ISSN: 0378-8741
- DATABASE BIOSIS [Online] BIOSCIENCES INFORMATION SERVICE, PHILADELPHIA, PA, US; 1986 BHATNAGAR S K ET AL: "EFFECTS OF 50 PERCENT ETHANOL EXTRACT OF CALOTROPIS-PROCERA AIT.F. ON ULCERS CAUSED BY ASSORTED TYPES OF CARCINOMA" Database accession no. PREV198784006510 XP002243509 & JOURNAL OF ECONOMIC AND TAXONOMIC BOTANY, vol. 8, no. 2, 1986, pages 489-490, ISSN: 0250-9768

## Description

### FIELD OF THE INVENTION

The present invention relates to the medical field. The invention relates in a first aspect to extracts of the plant *Calotropis procera,* having a pharmacological activity, in particular an anti-tumor activity and/or anti-poisonous activity and active compounds isolated thereof. In a second aspect, the present invention relates to methods for obtaining said extracts. The invention further relates in a third aspect to a pharmaceutical composition for the treatment of cancer comprising an effective amount of said extracts or an active compound thereof. In a fourth aspect, the present invention concerns the Use of said extracts or an active compound thereof as a medicament and the use of said extracts or an active compound thereof for the preparation of a medicament for the treatment of cancer.

### BACKGROUND OF THE INVENTION

Cancer develops in a given tissue when some genomic mutation perturbs cell cycle kinetics by increasing cell proliferation or decreasing cell death, or both. This perturbation leads to unrestrained growth of a genomically transformed cell population. Some cells from this transformed cell population may switch to the angiogenic phenotype, enabling them to recruit endothelial cells from the healthy tissue and leading to the sustained growth of the developing neoplastic tumor tissue. Subsequently, some cells migrate from the neoplastic tumor tissue and colonize new tissues, using blood or lymphatic vessels as major routes of migration. This process is also known as the metastatic process.

In practice, most of the agents used today in hospitals to treat cancer patients are drugs, which more or less directly target the cell kinetics, i.e. cell proliferation, of the cancer to be combated. The working mechanism of such anti-cancer drugs essentially relates to the disruption of the development of malignant cells by acting on cell kinetics. These drugs include alkylating agents, intercalating agents, antimetabolites, etc.., most of which target DNA or enzymes regulating the DNA duplication and elongation process. These drugs attack DNA.

A major drawback of these drugs involves that the drugs do not work in a selective manner, i.e. they do not select between normal and neoplastic cells. They are used in accordance with the fact that the DNA of rapidly proliferating cells, i.e. cancer cells, is more sensitive to this type of agents than the DNA of less rapidly proliferating cells, i.e. normal cells. However, rapidly growing tumors are not always tumors exhibiting high levels of cell proliferation. Rapidly growing tumors may also include tumors which exhibit low levels of cell death compared to the normal cell population from which these tumor cells issue. For these types of rapidly growing tumors, the mentioned drugs are not effective.

In addition, the great majority of the drugs used in the standard treatment of cancer using the cell kinetics approach have the drawback of being toxic or even highly toxic, i.e. involving many detrimental side-effects on healthy cells, tissues and organs, and this limits their clinical use to a relatively low number of administrations per patient. In addition, several of these compounds must be combined into a poly-chemotherapeutic regimen in order to have any observable effect against cancer. By way of evidence such anti-cancer drug combinations increase detrimentally the toxicity of the treatment and also limit the number of administrations that can be applied.

Some anti-cancer drugs from natural origins, such as e.g. anti-tubulin compounds, using a therapeutic approach different from the cell kinetics approach, have been proposed. Said drugs aim to prevent the migration of cancer cells which escape from the tumor bulk and first invade neighboring tissue therefore establishing metastases. However, the compounds of this type known so far also show major toxic side-effects, which limits their use over long periods of treatment.

Therefore, there remains an urgent need in the art for finding improved anti-cancer drugs, which overcome at least some of the above-mentioned drawbacks. Consequently, it is a general object of the invention to provide improved anti-cancer drugs. In particular the invention aims to provide an improved anti-cancer drug, showing minimal side effects.

### SUMMARY OF THE INVENTION

One embodiment of the present invention is an extract of the plant *Calotropis procera,* characterized in that said extract has a pharmacological activity, in particular an anti-poisonous activity. In a first aspect, the present invention therefore relates to the use of a composition comprising an extract of *Calotropis procera* according to claim 1.

In another aspect, the invention relates to an extraction process for obtaining an extract having biologically active components according to claim 12. In yet another aspect, the invention relates to an active extract according to claim 13.

An embodiment of the present invention is an extract as described above obtained using an extraction procedure, comprising the steps of:
a) extracting the starting material of said *Calotropis procera* plant, said starting material being selected among fruits, aerial parts subterranean parts, and their mixtures, in an aliphatic alcohol, by dissolving the starting material in said alcohol thereby obtaining a suspension of said material in said alcohol, stirring said suspension, and filtering said suspension by fritted glass thereby obtaining a first filtrate and a first solid part;
b) extracting said first solid part in an aliphatic alcohol thereby obtaining a second filtrate and a second solid part;
c) combining said first and said second filtrate thereby obtaining a combined filtrate, and evaporating said combined filtrate under vacuum thereby obtaining said extract.

Another embodiment of the present invention is an extract as described above obtained using a extraction procedure comprising the steps:
a) grinding the starting material of leaf blades, stems, barks and roots of *Calotropis procera* to give a fine powder of the plant,
b) extracting the powder of step a) with dichloromethane for at least 6, 12, 18 or preferably 24 hours using a soxhlet extractor,
c) decanting the dichloromethane of step b), and evaporating the filtrate, after filtration, to obtain a gum.

Another embodiment of the present invention is an extract as described above obtained using a extraction procedure comprising the steps:
a) grinding the starting material of leaf blades, stems, barks and roots of *Calotropis procera* give a fine powder of the plant,
b) extracting the powder of step a) with dichloromethane for at least 6, 12, 18 or preferably 24 hours using a soxhlet extractor,
c) decanting the dichloromethane of step b), and evaporating the filtrate, after filtration, to obtain a gum,
d) extracting the residue of step c) with methanol for at least 6, 12, 18 or preferably 24 hours using a soxhlet extractor,
e) decanting the methanol of step d), evaporating the filtrate, after filtration, to obtain a gum,
f) subjecting the gum of step e) to column chromatography using flash silica gel and dichloromethane-methanol as solvent, and
g) collecting a first fraction and evaporating of the said fraction to obtain a gum having biologically active components.

Another embodiment of the present invention is an extract as described above obtained using a extraction procedure comprising the steps:
a) grinding the starting material of leaf blades, stems, barks and roots of Calotropis procera give a fine powder of the plant,
b) extracting the powder of step a) with dichloromethane for at least 6, 12, 18 or preferably 24 hours using a soxhlet extractor,
c) decanting the dichloromethane of step b), and evaporating the filtrate, after filtration, to obtain a gum,
d) extracting the residue of step c) with methanol for at least 6, 12, 18 or preferably 24 hours using a soxhlet extractor,
e) decanting the methanol of step d), evaporating the filtrate, after filtration, to obtain a gum,
f) subjecting the gum of step e) to column chromatography using flash silica gel and dichloromethane-methanol as solvent,
g) collecting a first fraction, having biologically active components,
h) applying the concentrated fraction of step g) to column chromatography using flash silica gel and hexane-acetone as solvent to give two fractions, and
i) washing the column after step h) with methanol to give a third fraction, having biologically active components.

Another embodiment of the present invention is a composition comprising:
- an extract of *Calotropis procera* as described above, and
- at least one therapeutic compound and/or a physical treatment that exerts relevant, detrimental side effects on normal, non-cancer related cells, tissues or organs.

Another embodiment of the present invention is a product containing
- an extract of *Calotropis procera,* as described above, and
- at least one therapeutic compound and/or a physical treatment that exerts relevant, detrimental side effects on normal, non-cancer related cells, tissues or organs as a combined preparation for simultaneous, separate or sequential administration to a subject.

Another embodiment of the present invention is a composition as described above or a product as describe above where one of the said extracts comprises at least two active compounds selected from the group comprising asclepin, calactin, vorusharin, calotropin, calotropagenin, uzarigenin, calotoxin, usharin, -usharidin and 2' oxo-vorusharin.

Another embodiment of the present invention is a composition as described above, or a product as describe above wherein one of the said extracts comprises at least one of the compounds which are represented in Table 1.

Another embodiment of the present invention is a composition as described above or a product as describe above wherein the weight ratio of extract: therapeutic compound is in the range 0.001 : 1 to 1000 : 1.

Another embodiment of the present invention is a composition as described above, or a product as describe above, for use as a medicament.

Another embodiment of the present invention is a composition as described above, or a product as describe above, for use as a medicament for the treatment of cancer.

Another embodiment of the present invention is a composition or product as described above, wherein said cancer is selected from the group comprising breast cancer, lymphoma, sarcoma, pancreatic cancer, melanoma, colorectal cancer, glioma, non small cell lung cancer, small cell lung cancer, skin cancer, bone cancer, ovarian cancer, CNS cancer, renal cancer, bladder cancer, head and neck cancer, prostate cancer, liver cancer, hematological cancers.

Another embodiment of the present invention is a composition as described above or a product as describe above further comprising one or more additional therapeutic compounds.

Another embodiment of the present invention is a composition as described above, or a product as describe above, wherein said therapeutic compound(s) is an anti-cancer agent.

Another embodiment of the present invention is a composition as described above, or a product as describe above, wherein said therapeutic compound is selected from the group comprising adriamycin, alkeran, ara-c, bleomycin, biCNU (carmustine), busulfan, CCNU (lomustine), camptothecine, carboplatinum, cisplatinum, cyclophosphamide, cytoxan, daunorubicin, DTIC (dacarbazine), 5-FU (fluorouracil), fludarabine, gemcitabine (gemzar), herceptin, hexamethylmelamine, hydrea, idarubicin, ifosfamide, irinotecan (camptosar, CPT-11), leustatin, methotrexate, mithramycin, mitomycin, mitoxantrone, muphoran, navelbine, nitrogen mustard, oxaliplatine, rituxan, STI-571 (imatinib), streptozocine, taxol, taxotere, topotecan (hycamtin), velban, vincristine, VP-16 (etoposide), xeloda (capecitabine), or zevelin.

Another embodiment of the present invention is a composition as described above, or a product as describe above, wherein said therapeutic compound is selected from the group comprising adriamycine, vincristine, camptothecin and oxaliplatin.

Another embodiment of the present invention is a composition as described above, or a product as describe above, wherein said therapeutic compound(s) is a cytotoxic antibody or a fragment thereof.

Another embodiment of the present invention is a composition as described above, or a product as describe above, wherein said therapeutic compound(s) is a cytotoxic peptide or a fragment thereof.

Another embodiment of the present invention is a composition as described above further comprising a pharmaceutically acceptable carrier or a product as describe above wherein the composition and/or therapeutic compound(s) further comprises a pharmaceutically acceptable carrier.

Another embodiment of the present invention is a use of an extract of *Calotropis procera* as described above for the preparation of a medicament for alleviating the side effects of one or more therapeutic compounds.

Another embodiment of the present invention is a use of an extract of *Calotropis procera* as described above for the preparation of a medicament for increasing the dose administered to an individual of one or more therapeutic compounds.

Another embodiment of the present invention is a use of an extract as described above wherein said therapeutic compound(s) has anti-cancer activity.

Another embodiment of the present invention is a use of an extract as described above wherein said therapeutic compound(s) is selected from the group comprising adriamycin, alkeran, ara-c, bleomycin, biCNU, busulfan, CCNU, carboplatinum, cisplatinum, cyclophosphamide, cytoxan, daunorubicin, DTIC, 5-FU, fludarabine, gemcitabine (gemzar), herceptin, hexamethylmelamine, hydrea, idarubicin, ifosfamide, irinotecan (camptosar, CPT-11), leustatin, methotrexate, mithramycin, mitomycin, mitoxantrone, muphoran, navelbine, nitrogen mustard, oxaliplatine, rituxan, STI-571, streptozocine, taxol, taxotere, topotecan (hycamtin), velban, vincristine, VP-16, xeloda (capecitabine), or zevelin.

Another embodiment of the present invention is a use of an extract as described above wherein said therapeutic compound(s) is selected from the group comprising adriamycine, vincristine, camptothecin and oxaliplatin.

Another embodiment of the present invention is a use of an extract as described above wherein said therapeutic compound(s) is a cytotoxic antibody or a fragment thereof.

Another embodiment of the present invention is a use of an extract as described above wherein said therapeutic compound(s) is a cytotoxic hormone or a fragment thereof.

Another embodiment of the present invention is a use of an extract as described above wherein said therapeutic compound(s) is a cytotoxic peptide or a fragment thereof.

Another embodiment of the present invention is a use of an extract as described above wherein said extract comprises at least two active compounds selected from the group comprising asclepin, calactin, vorusharin, calotropin, calotropagenin, uzarigenin, calotoxin, usharin and usharidin.

Another embodiment of the present invention is a use of an extract as described above wherein said extract further contains at least one of the compounds which are represented in Table 1.

Another embodiment of the present invention is a use of an extract as described above wherein said extract is administered prior to said therapeutic compound(s).

Another embodiment of the present invention is a use of an extract as described above wherein said extract is administered after said therapeutic compound(s).

Another embodiment of the present invention is a use of an extract as described above wherein said extract is administered at the same time as said therapeutic compound(s).

Another embodiment of the present invention is a use of an extract as described above wherein the weight ratio of extract:therapeutic compound is in the range 0.001:1 to 1000:1.

Another embodiment of the present invention is an extraction process for obtaining an extract having biologically active components comprising the steps of:
a) extracting the starting material of said *Calotropis procera* plant, said starting material being selected among fruits, aerial parts, subterranean parts, and their mixtures, in an aliphatic alcohol, by dissolving the starting material in said alcohol thereby obtaining a suspension of said material in said alcohol, stirring said suspension; and filtering said suspension by fritted glass thereby obtaining a first filtrate and a first solid part;
b) extracting said first solid part in an aliphatic alcohol thereby obtaining a second filtrate and a second solid part;
c) combining said first and said second filtrate thereby obtaining a combined filtrate; and
d) evaporating said combined filtrate under vacuum thereby obtaining said extract.

Another embodiment of the present invention is an extraction process for obtaining a several extracts having biologically active components present substantially in the leaves, stems, barks and roots of *Calotropis procera,* which comprises the following steps:
a) grinding the starting material of leaf blades, stems, barks and roots of Calotropis procera give a fine powder of the plant,
b) extracting the powder of step a) with dichloromethane for at least 6, 12, 18 or preferably 24 hours using a soxhlet extractor, and
c) decanting the dichloromethane of step b), and evaporating the filtrate, after filtration, to obtain a gum.

Another embodiment of the present invention is an extraction process as described above further comprising the steps of:
d) extracting the residue of step c) with methanol for at least 6, 12, 18 or preferably 24 hours using a soxhlet extractor,
e) decanting the methanol of step d), evaporating the filtrate, after filtration, to obtain a gum,
f) subjecting the gum of step e) to column chromatography using flash silica gel and dichloromethane-methanol as solvent, and
g) collecting a first fraction having biologically active components,and evaporating the eluent to obtain said extract.

Another embodiment of the present invention is an extraction process as described above, further comprising the steps of
h) applying fraction of step g) to column chromatography using flash silica gel and hexane-acetone as solvent to give two fractions,
i) washing the column after step h) with methanol to give a third fraction, having biologically active components

Another embodiment of the present invention is a process as described above, wherein step b) is performed at a working temperature of between 20 and 80°C.

Another embodiment of the present invention is a process as described above, wherein said step b) is repeated between one and five times.

Another embodiment of the present invention is a process as described above, wherein the duration of step b) is between 4 hours and 48 hours.

Another embodiment of the present invention is a process as described above, wherein the solid phase of step d) is silica gel.

Another embodiment of the present invention is a process as described above, wherein the eluent of step d) is a binary eluent, the ratio between the two components of the eluent being between 100:0 to 0:100.

Another embodiment of the present invention is a process as described above, wherein the components of said binary eluent comprise an alcoholic solvent and a non polar solvent.

Another embodiment of the present invention is a process as described above, wherein step e) is performed at a working temperature of between 20 and 50°C.

Another embodiment of the present invention is a process as described above, wherein the solid phase of step f) is silica gel.

Another embodiment of the present invention is a process as described above, wherein the eluent of step f) is a binary eluent, the ratio between the two components of eluent being between 100:0 to 0:100.

Another embodiment of the present invention is a process as described above, wherein the components of the binary eluent are a non-polar and a more polar solvent.

Another embodiment of the present invention is a process as described above, wherein the ratio between the two components of eluent, non-polar:polar, is between 50:50 and 100:0.

Another embodiment of the present invention is a process as described above, wherein the solvent of step h) is methanol.

Another embodiment of the present invention is a process as described above, wherein step e) is performed at a working temperature of between 20 and 50°C.

Another embodiment of the present invention is an active extract isolated from the process as described above.

Another embodiment of the present invention is a use of an active extract as described above as a medicament.

Another embodiment of the present invention is a use of an active extract as described above for the preparation of a medicament in the treatment of cancer.

Another embodiment of the present invention is the use of a composition according to claim 1 for the preparation of a medicament for treating cancer, wherein said cancer is selected from the group comprising breast cancer, lymphoma, sarcoma, pancreatic cancer, melanoma, colorectal cancer, glioma, non small cell lung cancer, small cell lung cancer, skin cancer, bone cancer, ovarian cancer, CNS cancer, renal cancer, bladder cancer, head and neck cancer, prostate cancer, liver cancer, hematological cancers.

The present invention further relates to a kit according to claim 14. Another embodiment of the present invention is a kit comprising a container in which an extract of *Calotropis procera* as described above is present, and a container in which a therapeutic compound is present.

### DETAILED DESCRIPTION OF THE INVENTION

The present invention relates to extracts of the plant *Calotropis procera.* In a first embodiment the invention relates to extracts of the plant *Calotropis procera,* which has a pharmacological activity, in particular an anti-tumor activity and / or an anti-poisonous activity. Surprisingly, at least one of the extracts according to the invention combines an anti-tumor effect with an anti-poisonous activity.

According to the present invention the term "anti-tumor activity", refers to the *in vitro* as well as *in vivo* anti-tumor effects exerted by at least one of the extracts or isolated compounds thereof. The anti-tumor effects essentially include but are not limited to a dramatic decrease of cell growth and a pro-apoptotic effect. Importantly, the extracts according to the invention exhibits anti-tumor activity on a large number of cancer types, such as breast cancer, melanoma or lymphoma cancers amongst others.

Another feature of the extracts according to the invention encompasses their anti-poisonous activity. The term "anti-poisonous activity" refers to the ability of the extracts according to the invention or isolated compounds thereof to attenuate or reverse the effects of therapeutic compounds or treatments. A "therapeutic compound", as used herein, refers to a compound that exerts a relevant detrimental, toxic effect(s) on normal, i.e. non-cancer related cells, tissues or organs. The term therapeutic compound may thus also include a compound, drug, physical treatment or medicament, used to treat a particular disease that exerts detrimental toxic side effects. Such side effects are known to the skilled person and include, but are not limited to tiredness, nausea, vomiting, sores, mouth sores, dry skin, sensitive skin, hair loss, reduced red and white blood count. Such therapeutic compounds include, but are not limited to chemotherapy agents, antibodies and hormones.

Therapeutic compounds useful according to the invention include those used in chemotherapy or are cytotoxic. They include, but are not limited to any of adriamycin, alkeran, ara-c, bleomycin, biCNU, busulfan, CCNU, carboplatinum, cisplatinum, cyclophosphamide, cytoxan, daunorubicin, DTIC, 5-FU, fludarabine, gemcitabine (gemzar), herceptin, hexamethylmelamine, hydrea, idarubicin, ifosfamide, irinotecan (camptosar, CPT-11), leustatin, methotrexate, mithramycin, mitomycin, mitoxantrone, muphoran, navelbine, nitrogen mustard, oxaliplatine, rituxan, STI-571, streptozocine, taxol, taxotere, topotecan (hycamtin), velban, vincristine, VP-16, xeloda (capecitabine), or zevelin.

It is another aspect of the invention that a therapeutic agent comprises one or more anticancer agents which are derived from an extract of the present invention. Possible anti-cancerous compounds according to the invention are any extracted from *Calotropis procera* such as asclepin, calactin, voruscharin, calotropin, calotropagenin, uzarigenin, calotoxin, uscharin and uscharidin.

In another embodiment of the invention, the anti-cancerous compounds are any extracted from *Calotropis procera* such as 2"oxo-voruscharin, and derivatives thereof as shown in Table 2.

It is another aspect of the invention that a therapeutic agent is an extract of the invention. Said extract may be identical to an extract that has an anti-poisonous activity. Alternatively, said extract may be identical to an extract that has an anti-poisonous activity, with the exception of the absence of one or more active compounds from either extract. Alternatively, said extract may be identical to an extract that has an anti-poisonous activity, with the exception of the presence of one or more additional active compounds in either extract. Alternatively, said extract may be identical to an extract that has an anti-poisonous activity, with the exception of difference in the concentration of one or more active compounds in either extract.

It is another aspect of the invention that the therapeutic compound is an antibody, or a fragment thereof. Said antibody demonstrates cytotoxic activity. Examples of therapeutic antibodies include, but are not limited to HerceptinR, Prostascint, Rituximab, and Trastuzumab.

It is another aspect of the invention that the therapeutic compound is a hormone, or a fragment thereof. Said hormone demonstrates cytotoxic activity. Examples of therapeutic hormones include, but are not limited to busereline, fluoxymesterone, flutamide, formestane, norethandrolone, norethisterone, prednisolone, prednisone, and tamoxifene

It is another aspect of the invention that the therapeutic compound is a peptide, or a fragment thereof. Said peptide demonstrates cytotoxic activity.

By fragment in reference to a antibody, hormone or peptide means a peptide comprising a portion of the antibody, hormone or peptide that is capable of recognizing the target of the whole antibody, hormone or peptide. The fragment is also capable of recognizing the target of the whole antibody, hormone or peptide. The portion may comprise amino acid substitutions, deletions or insertions that do not substantially change the recognition of the target by portion. The number of substitutions, deletions or insertions may be less than 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, 60, 65, 70, 75, 80, 85, 90, 95, 100, 200, 300, 400, or 500.

It is another aspect of the invention, that the anti-poisionous activity of the extracts allows the administration of a higher dose of the therapeutic compound. By administering a higher dose of therapeutic compound, the cancer may be more quickly and effectively treated. An individual in receipt of a higher dose, in combination with an extract of the invention would benefit from reduced side-effects of the therapeutic compound.

It is another aspect of the invention that the anti-poisionous activity of the extracts allows the administration of a combination of therapeutic compounds. A combination therapy would target different aspects of the cancer simultaneously, so leading to a more effective and efficient treatment. By administering the combination in accordance with the invention, the patent would suffer less side effects and benefit from the effectiveness of the combination therapy.

In another embodiment, the present invention relates to the active compounds, isolated from the extracts. The term 'active compounds' or 'active components' of the extracts are used herein as synonyms, and refer to the compounds present in the extracts, which exhibit an activity that is similar to at least one of the above-defined activities of the extracts.

In yet another embodiment, the present invention relates to methods for obtaining the extracts of the plant *Calotropis procera.*

Due to the fact that at least one of the extracts according to the invention exert an anti-tumor activity the extracts according to the invention are particularly useful for the treatment of diseases such as cancer. Therefore, in another aspect, the present invention relates to pharmaceutical compositions comprising one of the above-described extracts or at least one active compound thereof.

Furthermore, since the extracts according to the invention exert an anti-poisonous activity as well, it is also particularly suitable to be combined with other therapeutic compounds, such as other medicaments, which show toxic side effects. Therefore, in another embodiment, the present invention relates to a pharmaceutical composition comprising one of the above-described extracts or at least one active compound thereof, and a second compound, which exerts a pharmacological activity having toxic side effects.

Furthermore, the present invention relates to the use of one of said extracts and/or at least one active compound thereof as a medicament. The present invention further relates to the use of one of the extracts or at least one active compound thereof in the preparation of a medicament for the treatment of diseases, in particular cancer.

Other objects and advantages of the present invention will become apparent from the following detailed description taken in conjunction with the accompanying drawings.

### FIGURES

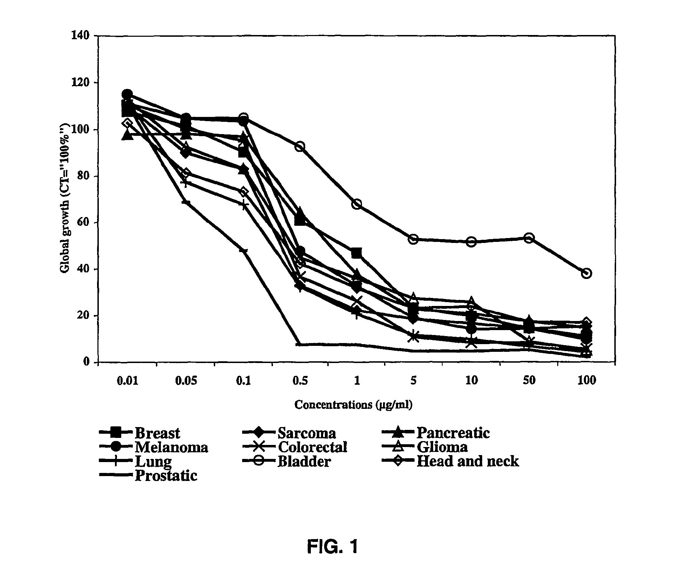

Figure 1 describes the overall growth of different types of tumors as a function of the concentration of an extract according to the invention.

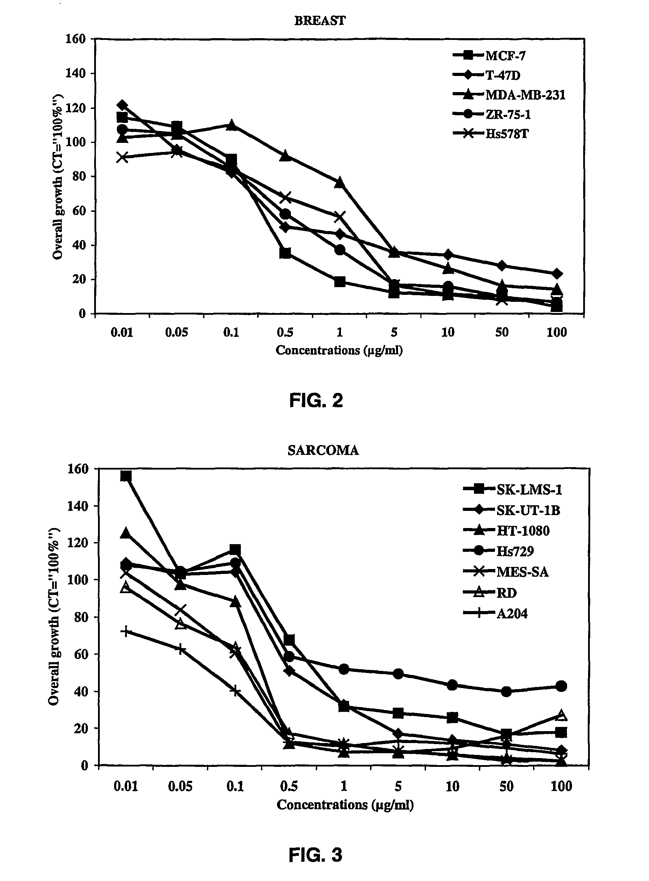

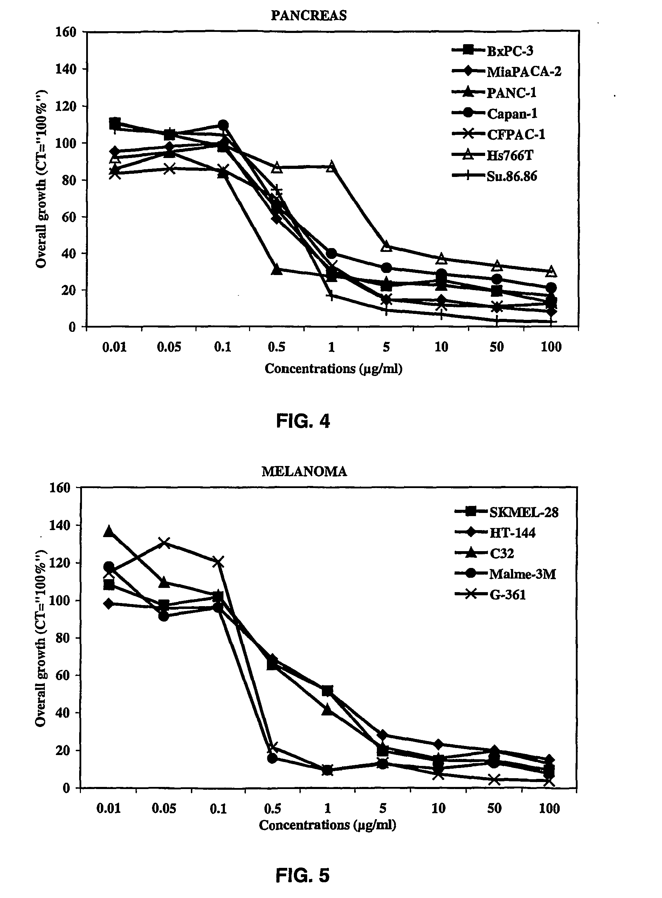

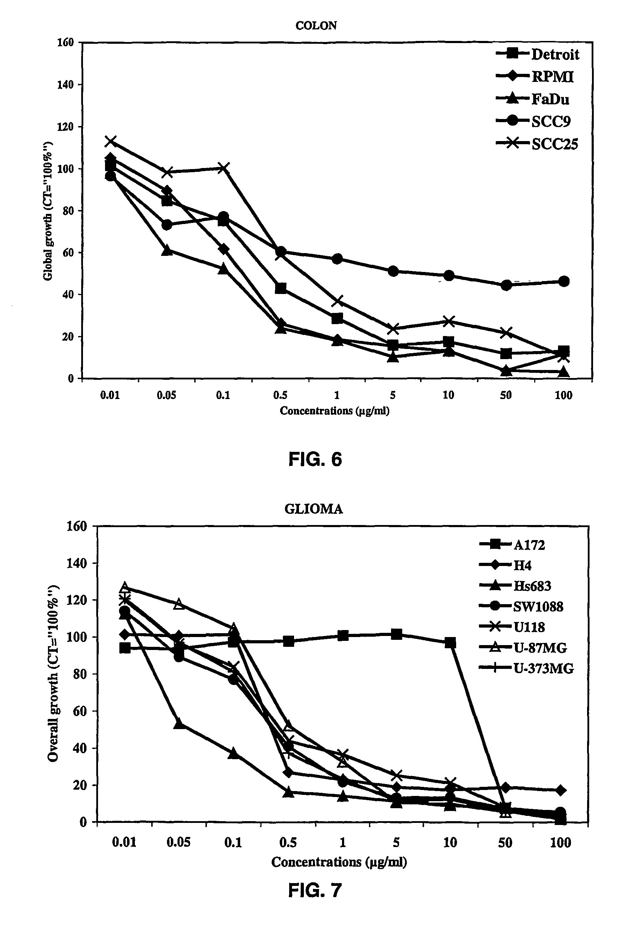

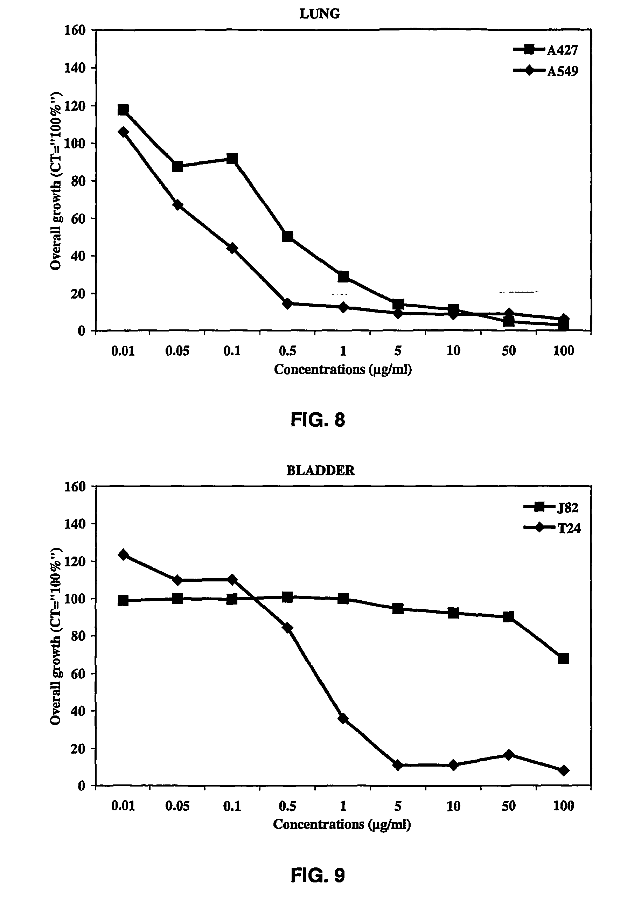

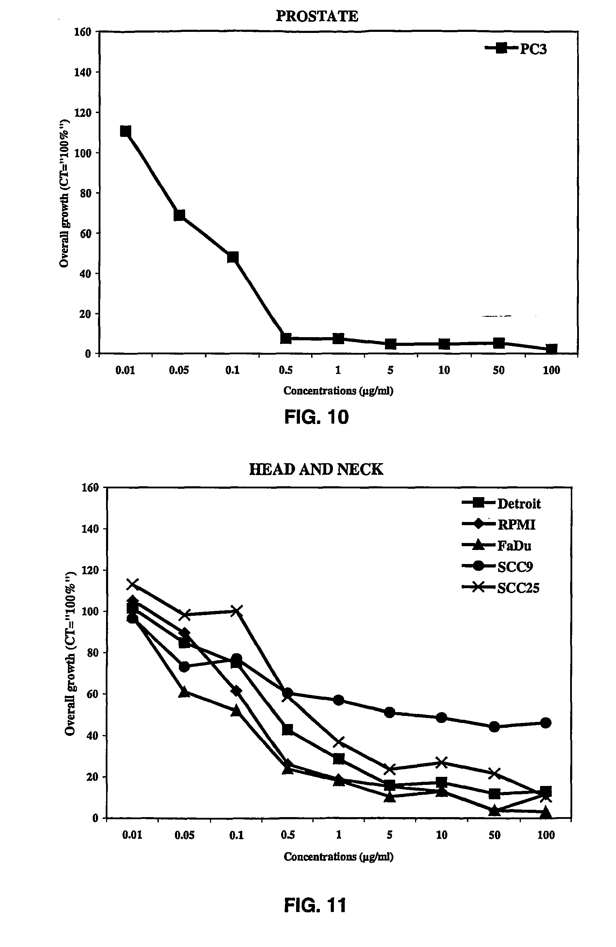

Figures 2 to 11 show the *in vitro* effects of an extract according to the invention on different types of human cancer cell lines, being breast cancer cell lines in Figure 2, sarcoma cancer cell lines in Figure 3, pancreatic cancer cell lines in Figure 4, melanoma cancer cell lines in Figure 5, colon cancer) cell lines in Figure 6, glioma cancer cell lines in Figure 7, lung cancer cell lines in Figure 8, bladder cancer cell lines in Figure 9, prostate cancer cell lines in Figure 10, and head and neck cancer cell lines in Figure 11.

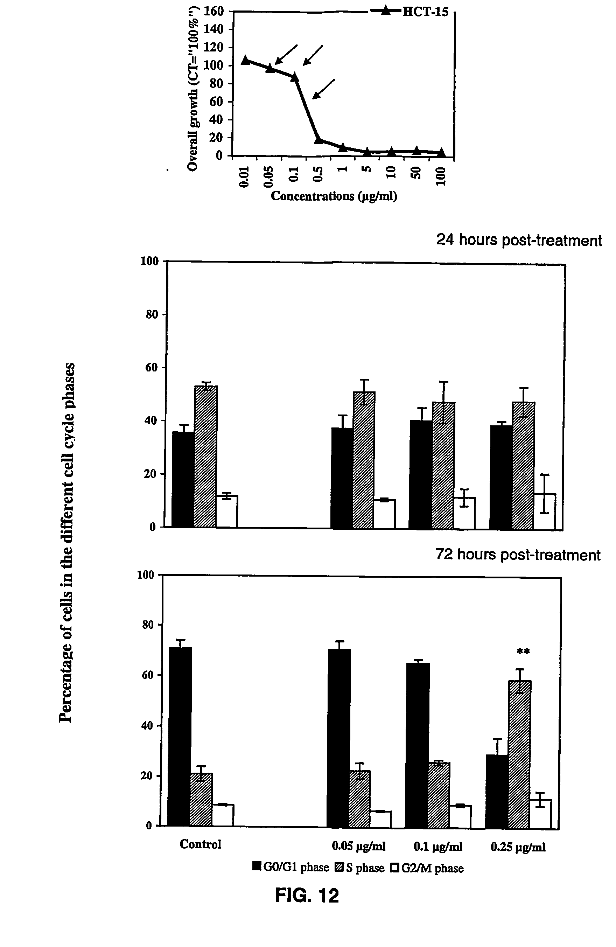

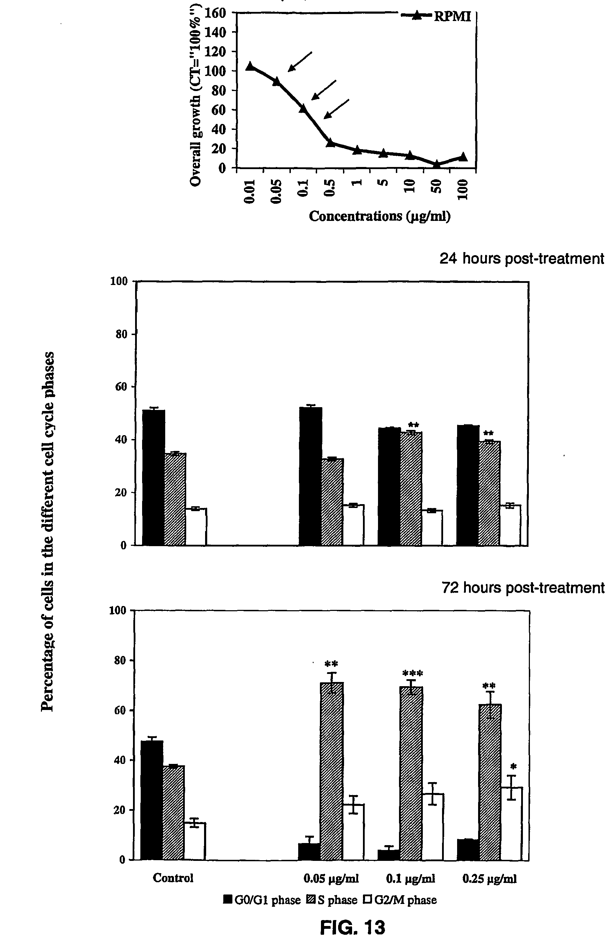

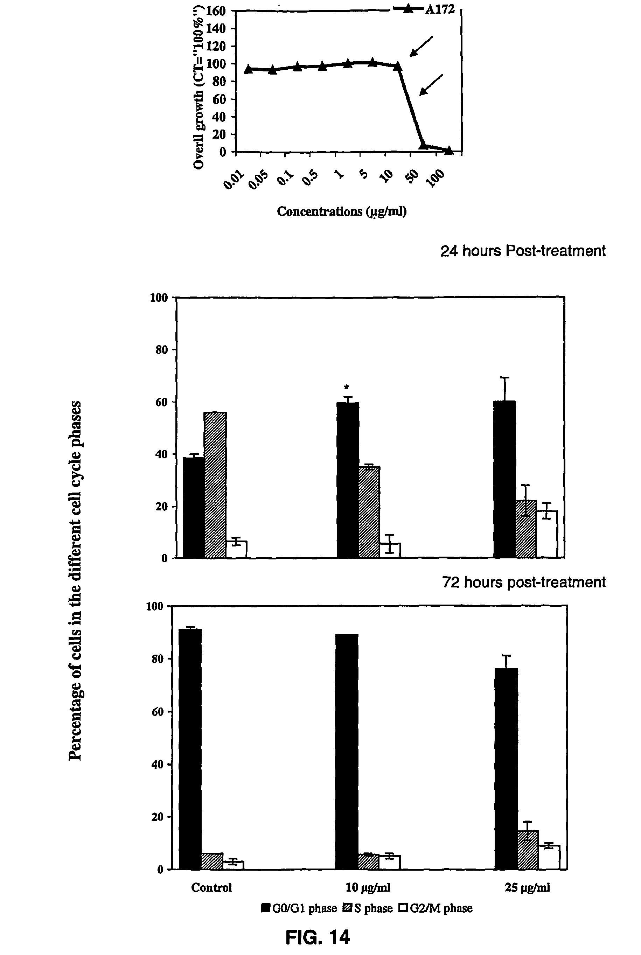

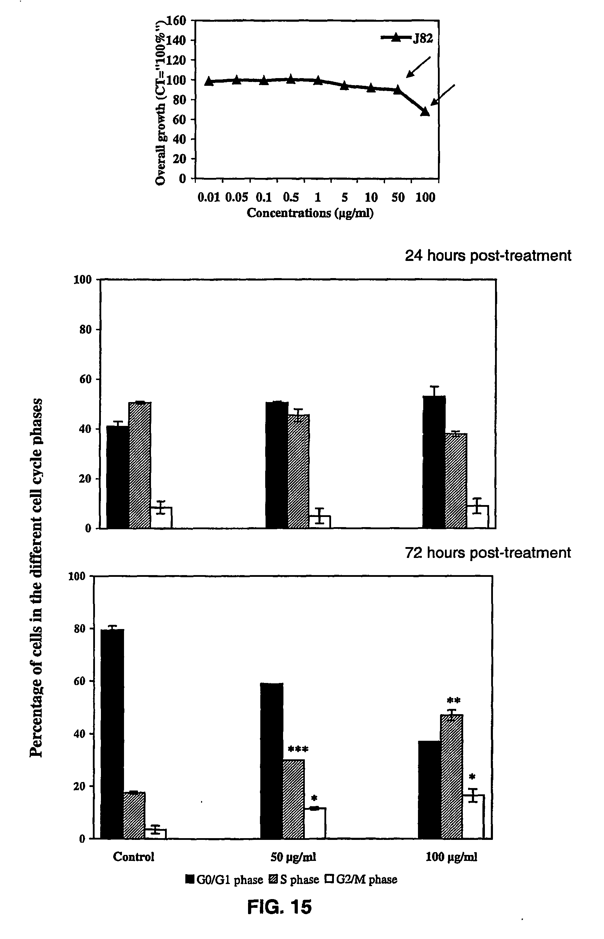

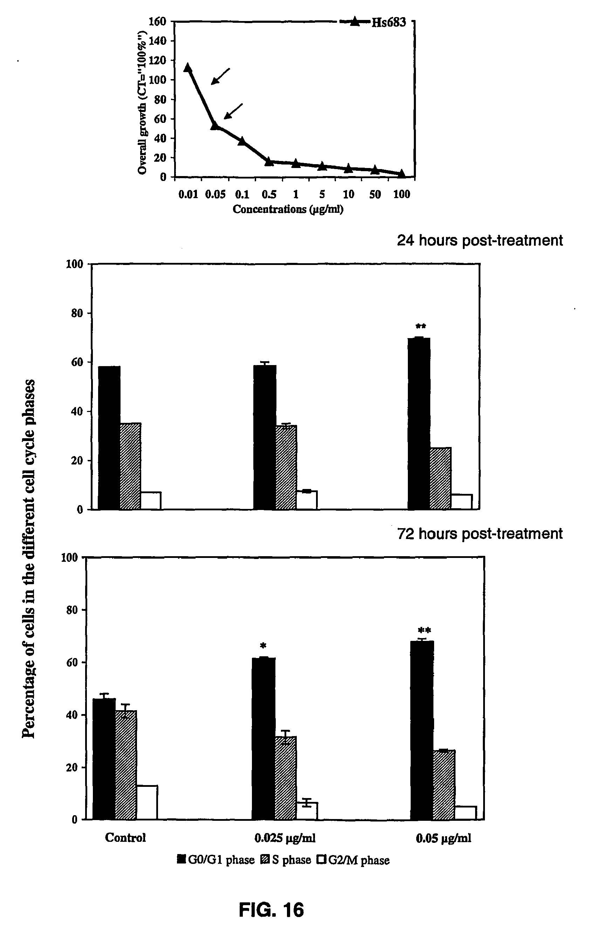

Figures 12 to 16 represent the *in vitro* effects of an extract according to the invention on the cell cycle kinetics of cells corresponding to five different human cell lines, HCT-15 (Figure 12), RPMI (Figure 13), A172 (Figure 14), J82 (Figure 15) and Hs683 (figure 16). The upper part of the figures shows overall cell growth, as the percentage of living cancer cells to control cells, at different doses of the extract. The middle and lower parts of the figures show the effect on the cell cycle kinetics of the said extract at different concentrations after 24 hours and 72 hours respectively. Statistical significance was evaluated with the Student's t-test where *: p<0.05; **: p<0.01: ***: p<0.001.

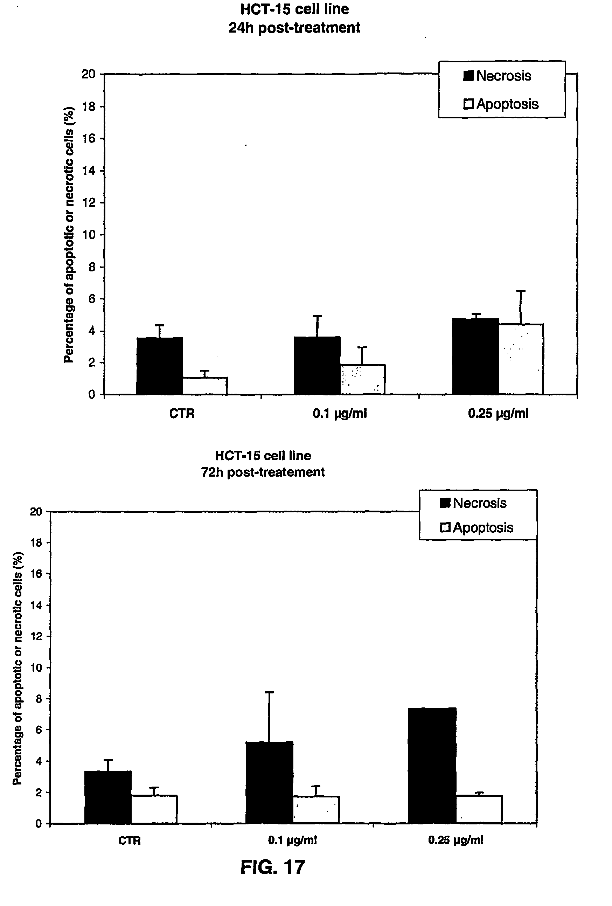

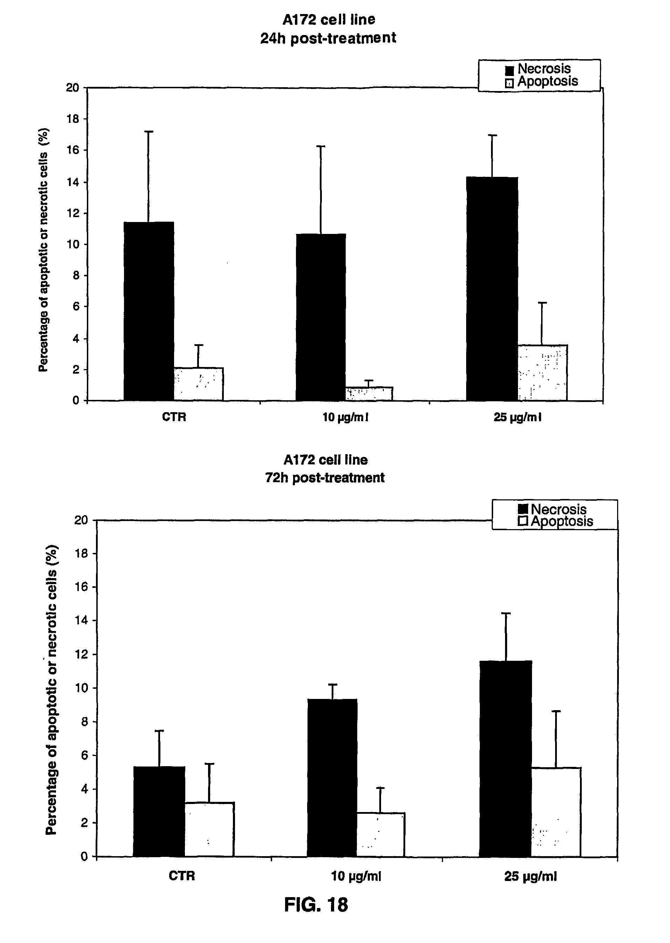

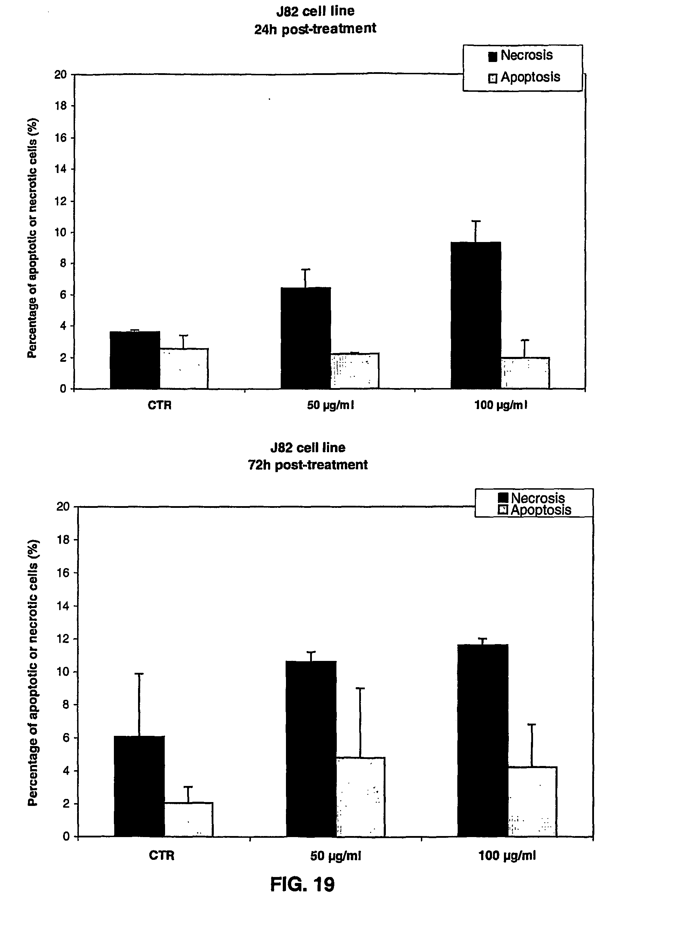

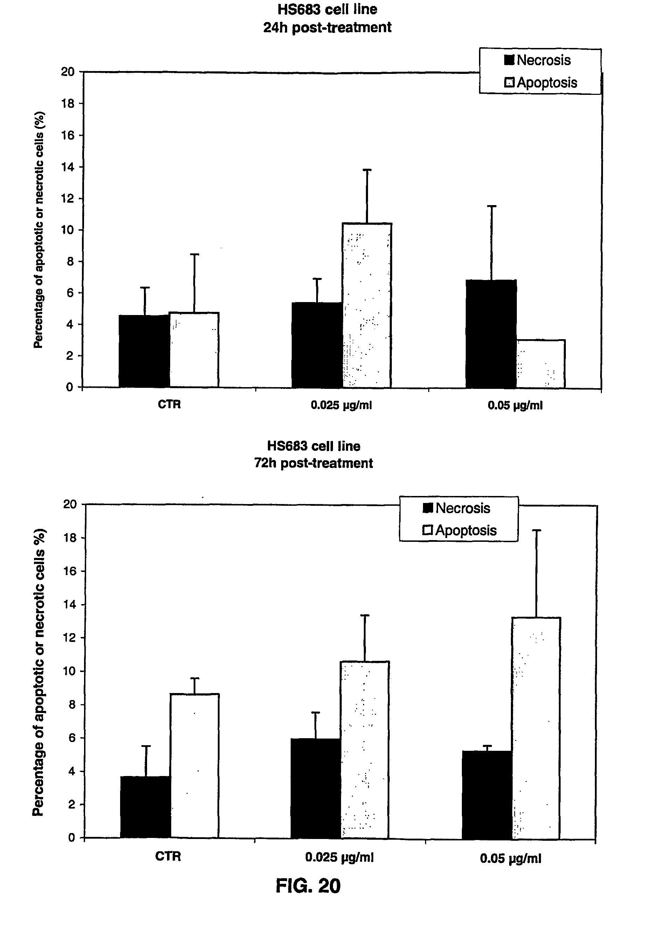

Figures 17 to 20 represent the *in vitro* apoptotic-inducing and necrosis-inducing effects of an extract according to the invention on cells corresponding to four different human cell lines, HCT-15 (figure 17), A172 (figure 18), J82 (figure 19) and Hs683 (figure 20). The upper part of the figures shows apoptotic-inducing and necrosis-inducing effects of the said extract at different doses after 24 hours, the lower part of the figures after 72 hours.

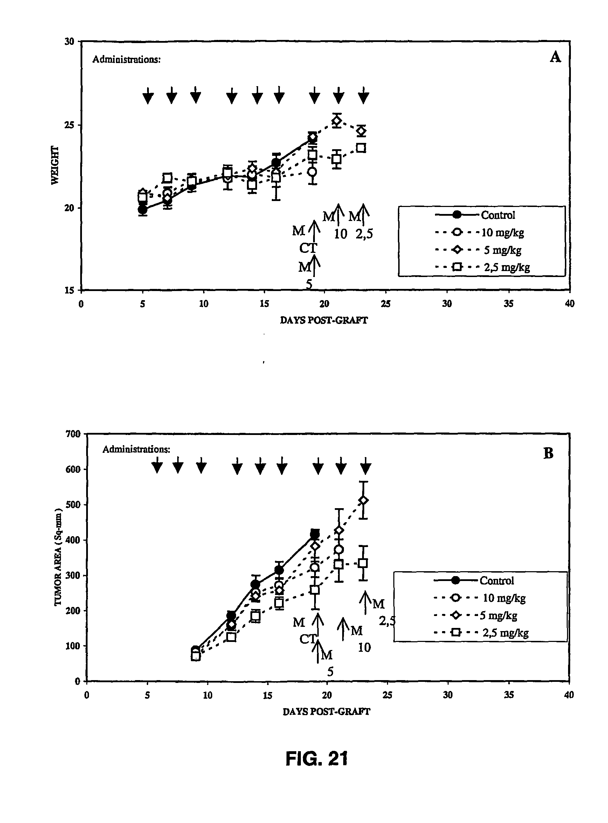

Figure 21 represents the effects of an extract according to the invention on an *in vivo* lymphoma cancer model. The said extract was administered by intra-peritoneal injection at doses of 2.5mg/kg; 5mg/kg and 10mg/kg extract in P388 lymphoma-tumor bearing mice. Figure 21A represents the effects of the said extract on the weight of control (untreated) mice and in treated mice. Figure 21 B represents the effects of the said extract on the tumor growth in control mice and in treated mice.

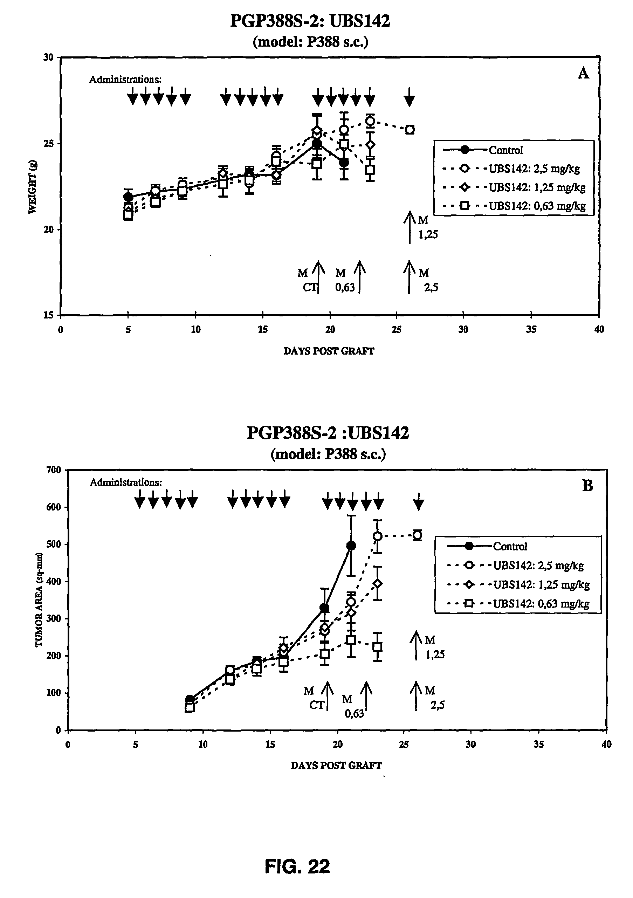

Figure 22 represents the effects of an extract according to the invention on an *in vivo* lymphoma cancer model. The said extract was administered by intra-peritoneal injection at doses of 0.63mg/kg; 1.25mg/kg and 2.5mg/kg extract in P388 lymphoma-tumor bearing mice. Figure 22A represents the effects of the said extract on the weight of control mice and treated mice. Figure 22B represents the effects of the said extract on the tumor growth in control mice and in treated mice

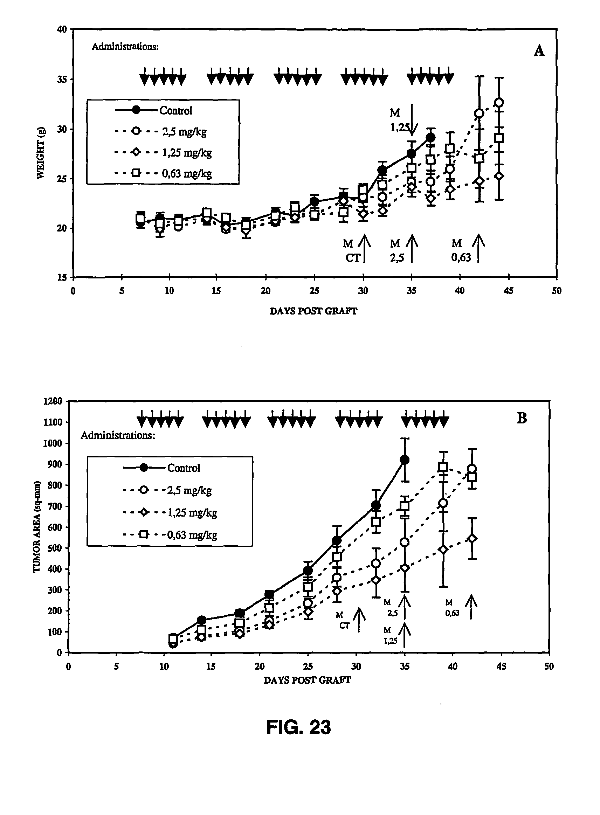

Figure 23 represents the effects of an extract according to the invention on an *in vivo* melanoma cancer model. The said extract was administered by intra-peritoneal at doses of 2.5mg/kg; 1.25mg/kg and 0.63mg/kg extract in B16-melanoma bearing mice. Figure 23A represents the effects of the said extract on the weight of control (untreated) mice and treated mice. Figure 23B represents the effects of the said extract on the tumor growth in control mice and in treated mice.

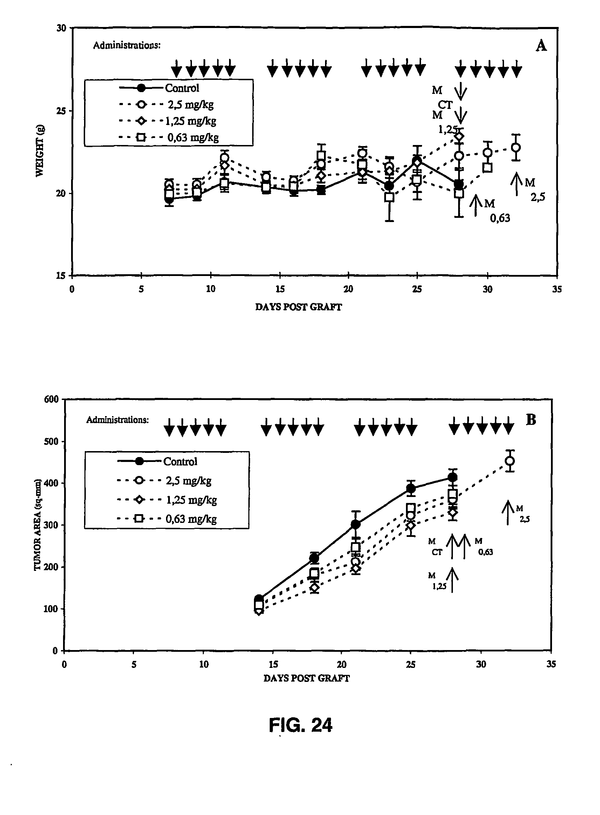

Figure 24 represents the effects of an extract according to the invention on an *in vivo* melanoma cancer model. The extract was administered per os at doses of 0.63mg/kg; 1.25mg/kg and 2.5mg/kg extract in B16-melanoma bearing mice. Figure 24A represents the effects of the said extract on the weight of control mice and treated mice. Figure 24B represents the effects of the said extract on the tumor growth in control mice and in treated mice.

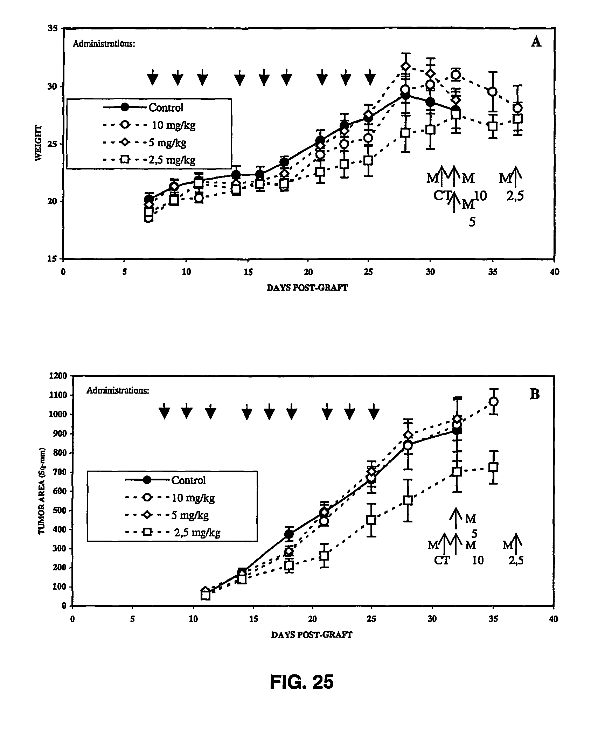

Figure 25 represents the effects of an extract according to the invention on an *in vivo* breast cancer model. The said extract was administered by intra-peritoneal injection at doses of 2.5mg/kg; 5mg/kg and 10 mg/kg extract in MXT-HI breast cancer bearing mice. Figure 25A represents the effects of the said extract on the weight of control (untreated) mice and treated mice. Figure 25B represents the effects of the said extract on the tumor growth in control mice and treated mice.

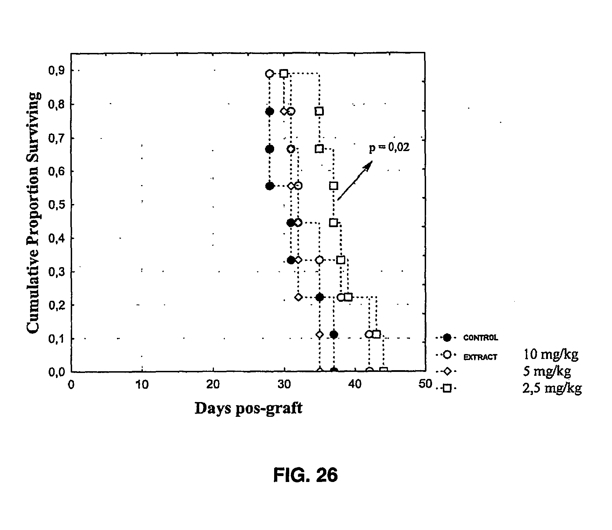

Figure 26 represents the statistical general fit for the MXT-HI breast cancer bearing mice of the test groups (i.e. intra-peritoneal injection of the mice at extract doses of 2.5mg/kg; 5mg/kg and 10 mg/kg) and the control group, with regard to survival (Kaplan-Meier statistical analysis).

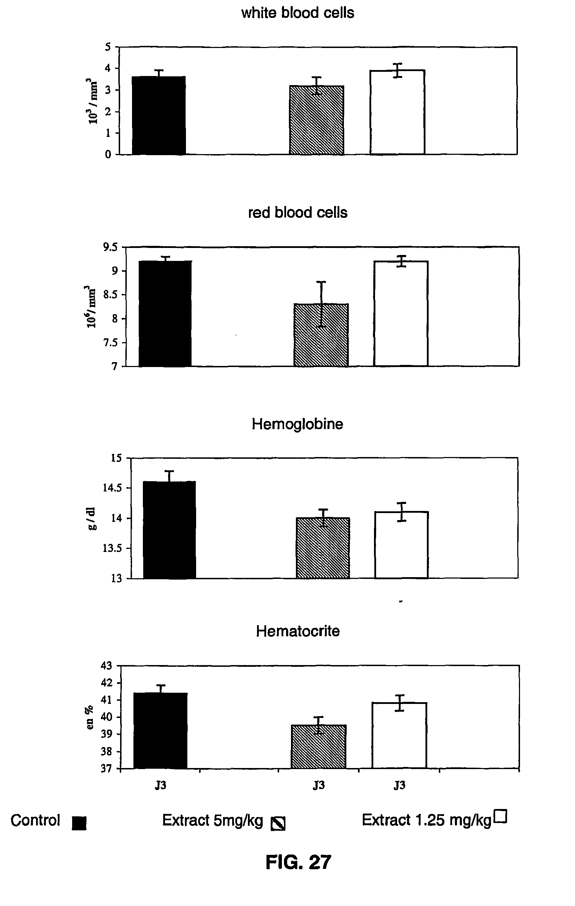

Figure 27 represents the effects of an extract according to the invention at doses of 5mg/kg and 1.25 mg/kg on the amount of white blood cell, red blood cell, the hemoglobin and hematocrite concentration at day three after intra-peritoneal injection in mice compared to control mice (In the Figure the "white" extract dose is 1,25 mg/kg

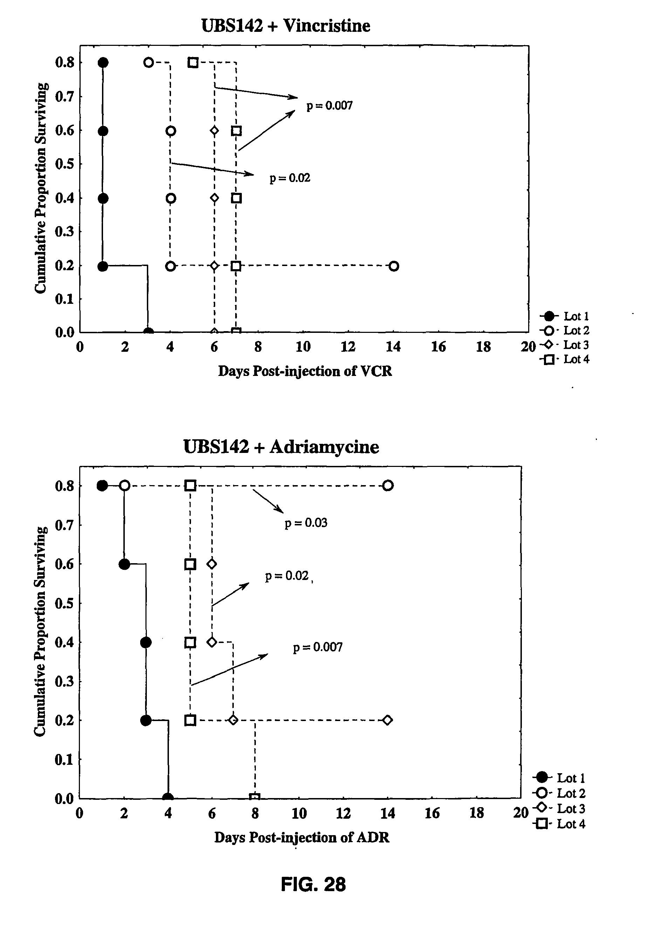

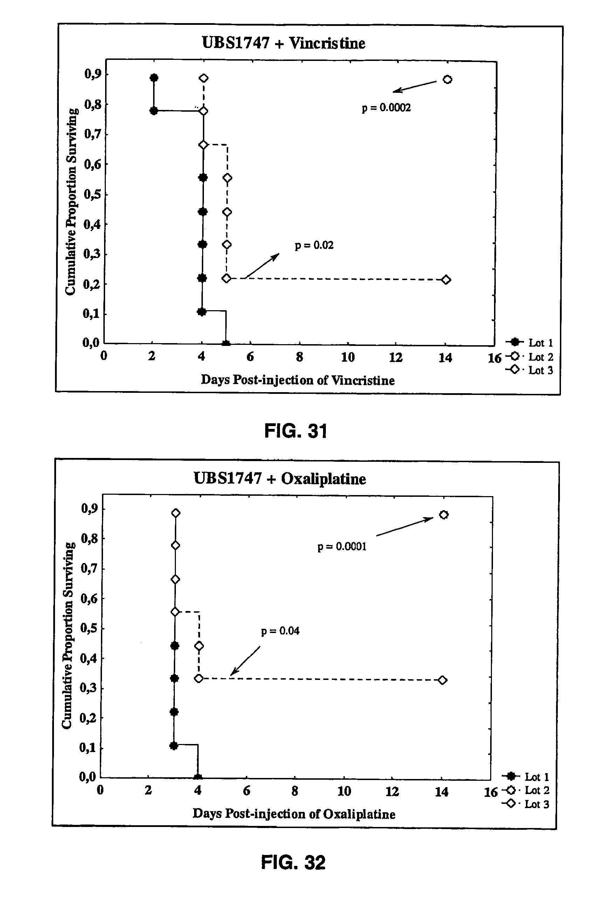

Figures 28 to 35 illustrate the anti-poisonous effects of extracts according to the invention when combined with anti-tumors compounds adriamycine or vincristine or camptothecin or oxaliplatin.

### Properties of the extract

The *Calotropis procera* plant, belonging to the family of the *Asclepiadaceae,* is a plant growing in Africa and Asia. This plant is used in traditional folk medicine and has also been studied with respect to a considerable number of uses such as an anti-pyretic, anti-malarial, anti-diarroeal, analgesic, anti-inflammtory gastric, mucosal protector and as an insecticidal, anti-tussive, antibacterial, wound healing, muscle relaxant. The stems, flowers and leaves of *Calotropis procera* plant are known to contain certain compounds known as cardenolides. Recently, cardiotoxic activity has been attributed to these cardenolides, and they are exploited in human therapies for treating cardiac insufficiencies.

Unexpectedly, the present invention relates to at least one extract according to the invention of the *Calotropis procera* plant showing another type of activity, in particular an "anti-tumor activity". Moreover, it was demonstrated that at least one extract according to the invention of shows *in vitro* as well as *in vivo* anti-tumor effects. The said extract according to the invention induces a dramatic reduction in cell growth and shows a pro-apoptotic activity. These properties are further illustrated in the examples 3 and 4. In addition, it has been in-vivo shown by the inventors that at least one of the said extracts according to the invention is highly active against several types of cancers. As is shown in the examples described below, the said extract according to the invention, exerts significant anti-tumor effects on several tumor models tested, which represent a broad panel of histological tumor types, including breast cancer, lymphomas, melanomas. These models are clinically relevant because they mimic specific clinical stages of human cancers.

Surprisingly, the said extract according to the invention is highly active at relatively low doses. The said extract can exert an anti-tumor activity at low doses in the range of 0.4 to 2.2 mg/kg and preferably in the range of 0.5 to 2 mg/kg. The high anti-tumor activity at low doses indicates that the said extract is highly active. Surprisingly, as will also become clear when reading the examples given below, the said extract according to the invention shows significantly higher anti-tumor effects when assayed at chronically low doses, i.e. 0.6 mg/kg to 1.25 mg/kg, than at high doses, i.e. at doses of 5 mg/kg to 10 mg/kg. The Maximum Tolerated Dose (MTD) index of the said extract according to the invention, referring to the maximum amount of the said extract, which can be administered acutely to healthy animals, is 20mg/kg extract. This value indicates that the said extract according to the invention has a broad therapeutic window.

The terms "toxicity" or 'toxic effects" relate to the detrimental effect(s) a compound may have on healthy cells, tissues or organs. Another important property of an extract according to the invention includes its low toxicity level. It was demonstrated that the said extract does not induce *in vivo* hematological changes, does not induce weight loss and shows minor side effects on different types of organs and tissues. In fact, the toxicity level of the said extract according to the invention is surprisingly low. The said extract according to the invention combines the essential features of a good anti-tumor activity, a low level of toxicity and a minimal induction of detrimental side effects.

Furthermore, the extracts of the plant *Calotropis procera* according to the invention also show an "anti-poisonous activity". As mentioned above, the term "anti-poisonous activity", as used herein, refers to the ability of the extracts according to the invention to attenuate or reverse the detrimental side effects of compounds. Surprisingly, the inventors have demonstrated that combination of an extract according to the invention with other therapeutic compounds, having detrimental side effects enables undesirable side effects of the therapeutic compound to be reduced. For instance, example 8 illustrates that the combination of an extract according to the invention with well known anti-cancer compounds, such as vincristine or adriamycine, camptothecin or oxaliplatin, enables the reduction of some side effects caused by the toxicity to normal cells induced by these anti-cancer compounds.

The anti-poisonous activity is evident when at least one of the extracts according to the invention is administered prior to the therapeutic compound *i.e.* they are administered sequentially. It is an aspect of the invention to administer an extract prior any time before the therapeutic compound is administered. It is a further aspect of the invention to administer an extract no more than 336, 312, 288, 264, 240, 216, 192, 168, 144, 120, 96, 72, 48, 24, 20, 16, 12, 8, 4, 2, 1, or 0.5 hours before the therapeutic compound is administered.

The anti-poisonous activity is also evident when at least one of the extracts according to the invention is administered at the same time as the therapeutic compound *i.e.* as a composition, by simultaneous or separate administration.

The anti-poisonous activity is also evident when at least one of the extracts according to the invention is administered after the therapeutic compound *i.e.* they are administered sequentially. It is an aspect of the invention to administer an extract any time after the therapeutic compound has been administered. It is a further aspect of the invention to administer the extract less than 0.5, 1, 2, 4, 8, 12, 16, 20, 24, 48, 72, 96, 120, 144, 168, 192, 216, 240, 264, 288, 312, or 366 hours after the therapeutic compound has been administered.

In sequential administration, the said extracts may be administered once, or any number of times and in various doses before and/or after administration of the therapeutic compound. Sequential administration may be combined with simultaneous or sequential administration.

### Composition of the extract

The extracts according to the invention comprise one or several active compounds, i.e. compounds present in an extract according to the invention, which exhibit an activity similar to at least one of the above-defined activities of an extract according to the invention. The main activities of at least one of the extracts are an anti-tumor activity and an anti-poisonous activity. As it will be understood by the one skilled in the art, an active compound present in the said extracts may exerts only one of these properties, or even both of these properties.

In another embodiment, at least one of the extracts according to the invention comprise at least two of the active compounds selected from the group comprising asclepin, calactin, voruscharin, calotropin, calotropagenin, uzarigenin, calotoxin, uscharin and uscharidin, 2"oxo voruscharin.

As it will be understood, the extracts according to the invention may further contain one or more active compounds, which do not belong to the group of the cardenolides, and other compounds. In another embodiment, the extracts contain at least one of the compounds represented in Table 1.

**TABLE 1 Some of the compounds contained in at least one of the extracts according to the invention**

| | |
|---|---|
| Compounds present in the extract according to the invention Invention | 3-O-ACETYL-CALOTROPIN, ALPHA-AMYRIN, ALPHA-AMYRIN-BENZOATE, ALPHA-CALOTROPEOL, ALPHA-LACTUCEROL, ALPHA-LACTUCERYL-ACETATE, ALPHA-LACTUCERYL-ISOVALERATE, ARABINOSE, ASH, BENZOYLISOLINEOLONE, BENZOYLLINEOLONE, BETA-AMYRIN, BETA-AMYRIN-BENZOATE, BETA-CALOTROPEOL, BETA-SITOSTEROL, CAOUTCHOUC, COROGLAUCOGENIN; COROTOXIGENIN; D-GLUCOSAMINE, FRUGOSIDE, GIGANTEOL, GIGANTIN, GLUCOSE, HISTAMINE, ISOGIGANTEOL, ISOLACTUCEROL, ISOLINEOLONE, LAURANE LINEOLONE, LINOLEIC-ACID, LINOLENIC-ACID, MELISSYL-ALCOHOL, MUDARINE, OLEIC-ACID, PALMITIC-ACID, PROCEROSIDE, PSEUDOCALOTROPAGENIN, RHAMNOSE STIGMASTEROL, SYRIOGENIN TARAXASTEROL, TARAXASTEROL-BENZOATE, TRYPSIN |

In another embodiment, the present invention relates to an active compound isolated from the extracts according to the invention.

In another embodiment of the invention, an active compound of the extract is 2"oxo-voruscharin. An aspect of the invention is the anti-cancer and/or anti-poisonous activity of said compound (compound A) and the derivatives (compounds B to H) thereof as shown in Table 2.

**TABLE 2: Chemical structure of 2"oxo-voruscharin (Compound A) and derivatives there of (Compounds B to H).**

| | 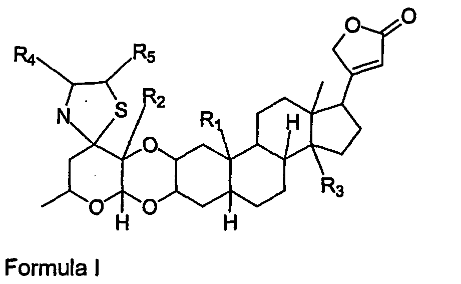 | | | | |
|---|---|---|---|---|---|
| | **R₁** | **R₂** | **R₃** | **R**₄ | **R₅** |
| **Compound A** 2"oxo-voruscharin | -COH | -OH | -OH | =O | -H |
| **Compound B** | -CH₂OH | -OH | -OH | =O | -H |
| **Compound C** | -CH₂OAc | -OH | -OH | =O | -H |
| **Compound D** | -CH₂OOCphenyl | -OH | -OH | =O | -H |
| **Compound E** | -CH₂OH | -OH | -OH | Double bond* | -H |
| **Compound F** | -CH₂OAc | -OH | -OH | Double bond* | -H |
| **Compound G** | - CH₂OOCphenyl | -OH | -OH | Double bond* | -H |
| **Compound H** | -CH₂OH | -OH | -OH | 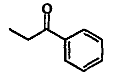 | -H |

| | | | | | |
|---|---|---|---|---|---|
| * means a double bond between the N atom and the C carbon atom of the N-containing heterocyclic ring of formula I. | | | | | |

### Methods of extraction

According to another embodiment, an extract according to the invention can be obtained by an alcoholic extraction, in particular by a methanol extraction.

In another embodiment, the present invention relates to a method for obtaining an extract according to the invention comprising the steps of:
a) extracting the starting material of said *Calotropis procera* plant, said starting material being selected among fruits, aerial parts, subterranean parts, and their mixtures, in an aliphatic alcohol, by dissolving the starting material in said alcohol thereby obtaining a suspension of said material in said alcohol, stirring said suspension; and filtering said suspension by fritted glass thereby obtaining a first filtrate and a first solid part;
b) extracting said first solid part in an aliphatic alcohol thereby obtaining a second filtrate and a second solid part;
c) combining said first and said second filtrate thereby obtaining a combined filtrate; and
d) evaporating said combined filtrate under vacuum thereby obtaining an oily residue.

In a preferred embodiment, the starting material of said *Calotropis procera* plant is selected from subterranean parts, in particular from roots.

In another embodiment, the aliphatic alcohol used to extract the starting material according to the invention is methanol. In yet another embodiment, the residue obtained in the extraction process is taken up in a solvent, in particular in a pharmaceutically acceptable solvent.

In another embodiment, the present invention relates to a method for obtaining extracts according to the invention, comprising the steps of:
a) Grinding the starting material of leaf blades, stems, barks and roots of *Calotropis procera* give a fine powder of the plant.
b) Extracting the powder of step a) with dichloromethane for at least 6, 12, 18 or preferably 24 hours using a soxhlet extractor
c) decanting the dichloromethane of step b), and evaporating the filtrate, after filtration, to form a gum
d) extracting the residue of step c) with methanol for at least 6, 12, 18 or preferably 24 hours using a soxhlet extractor
e) decanting the methanol of step d), evaporating the filtrate, after filtration, to form a gum
f) subjecting the gum of step e) to column chromatography using flash silica gel and dichloromethane-methanol as solvent
g) collecting a first fraction,
h) applying the concentrated fraction of step g) to column chromatography using flash silica gel and hexane-acetone as solvent to give two fractions,
i) washing the column after step h) with methanol to give a third fraction,

Extracts according to the present invention include those obtained from step c), step g), and step i).

It is an aspect of the invention that the extractions are performed in a manner compatible with preserving the integrity of the biologically active compounds contained in said extract. Such precautions are known to the skilled person.

### Applicability of the extracts

Due to the interesting properties of at least one of the extracts disclosed herein, in particular its anti-tumor activity, its anti-poisonous activity, its low level of toxicity and/or its high activity at low doses, the extract(s) according to the invention or active compounds thereof are particularly suitable for use as a medicament for the treatment of diseases, and in particular for treating cancer.

In another embodiment, the invention relates to the use of an extract according to the invention, or an active compound thereof, as a medicament.

In yet another embodiment, the invention also relates to the use of at least one extract according to the invention, or an active compound thereof, for the preparation of a medicament in the treatment of cancer. In particular the said extract according to the invention, or an active compound thereof is used for the preparation of a medicament in the treatment of a cancers. Examples of cancers that may be treated according to the invention include, but are not limited to breast cancer, lymphoma, sarcoma, pancreatic cancer, melanoma, colorectal cancer, glioma, non small cell lung cancer, small cell lung cancer, skin cancer, bone cancer, ovarian cancer, CNS cancer, renal cancer, bladder cancer, head and neck cancer, prostate cancer, liver cancer and hematological cancer.

As mentioned above, the extracts according to the invention also comprise an anti-poisonous activity. The inventors have demonstrated that a combination of an extract according to the invention or active compounds thereof with a second compound having relevant detrimental side effects enables a reduction of the toxic activity of this second compound, without reducing its therapeutic activity. Therefore, the *Calotropis procera* extracts according to the invention may also be combined in a medicament with other compounds, in particular with other anti-cancer compounds.

### Pharmaceutical compositions comprising the extract

In another embodiment, the present invention relates to a pharmaceutical composition for the treatment of cancer comprising a therapeutic effective amount of an extract of *Calotropis procera* according to the invention or an active compound thereof, and a pharmaceutical acceptable carrier.

In another embodiment, the present invention relates to a pharmaceutical composition for the treatment of cancer comprising
- a therapeutic effective amount of an extract of *Calotropis procera* according to the invention or an active compound thereof,
- a therapeutic effective amount of a therapeutic compound, and,
- a pharmaceutical acceptable carrier.

The term "therapeutically effective amount" as used herein means that amount of at least one extract or active compound or pharmaceutical agent that elicits the biological or medicinal response in a tissue, system, animal or human that is being sought by a researcher, veterinarian, medical doctor or other clinician, which includes alleviation of the symptoms of the disease being treated.

The pharmaceutical composition can be prepared in a manner known to one of skill in the art. For this purpose, an extract according to the invention and/or any active compound thereof, one or more solid or liquid pharmaceutical carriers and, if desired, in combination with other pharmaceutical active compounds, are brought into a suitable administration form or dosage form which can then be used as a pharmaceutical in human medicine or veterinary medicine.

Particular forms of the pharmaceutical composition may be, for example, solutions, suspensions, emulsions, creams, tablets, capsules, nasal sprays, lipsomes or micro-reservoirs, especially compositions in orally ingestible or sterile injectable form, for example, as sterile injectable aqueous or oleaginous suspensions or suppositories. The preferred form of composition contemplated is the dry solid form, which includes capsules, granules, tablets, pills, boluses and powders. The solid carrier may comprise one or more carriers, e.g. lactose, fillers, disintegrating agents, binders, e.g. cellulose, carboxymethylcellulose or starch or anti-stick agents, e.g. magnesium stearate, to prevent tablets from adhering to tabletting equipment. Tablets, pills and boluses may be formed so as to disintegrate rapidly or to provide slow release of the active ingredient.

Furthermore, due to their anti-poisonous activity, the extracts according to the invention or active compounds thereof, are also particularly suitable to be combined with other therapeutic compounds which exert a pharmacological activity having toxic side effects, such as other medicaments, which show toxic side effects.

The "therapeutic compound, which exerts a pharmacological activity having toxic side effects" may include any compound that is used for the treatment of any disease but which induces unwanted toxic effects. Preferably, such compounds are compounds that are used in the treatment of cancer. For instance, an extract according to the invention or active compounds thereof can be combined with one or more than one other compound that has an anti-tumor effect. Since two or more compounds having an anti-tumor effect are combined, an improved anti-tumor activity can be obtained. In addition, such combinations also enable to reduce the toxic side effects, which are induced by one or several of the anti-tumor compounds.

A combination of an extract according to the invention or active compounds thereof with another therapeutic compound may involve a separate use of the said extract or active compound thereof and the other therapeutic compound. In particular, the combination may involve the use of an extract according to the invention or active compounds thereof prior (pre-treatment) to, at the same time, or after (post-treatment) the use of the other therapeutic compound.

Alternatively, a combination of an extract or active compounds thereof with another therapeutic compound may also involve a mixture of both elements. Therefore, in a preferred embodiment, the present invention relates to a pharmaceutical composition comprising a first component, said first component being the above-described extract or at least one active compound thereof, and a second component, said second component comprising a therapeutic compound, which exerts a pharmacological activity having toxic side effects.

### Product

One aspect of the invention is a product containing an extract of *Calotropis procera* and a therapeutic compound for simultaneous, separate or sequential administration to a subject. It is an aspect of the invention that the product may be used according to the invention.

By simultaneous administration means the two components are administered to a subject at the same time. For example, as a mixture of the two components. Examples include, but are not limited to a solution administered intraveneously, a tablet, liquid, topical cream, etc.,
wherein each preparation comprises both components.

By separate administration means the two components are administered to a subject at the same time or substantially the same time, but the components are present in the product as separate, unmixed preparations. For example, the two components may be present in the product as individual tablets. The tablets may be administered to the subject by swallowing both tablets at the same time, or one tablet directly following the other.

By sequential administration means the two components are administered to a subject sequentially and the components are present in the product as separate, unmixed preparations. There is a time interval between doses. For example, one component might be administered up to 336, 312, 288, 264, 240, 216, 192, 168, 144, 120, 96, 72, 48, 24, 20, 16, 12, 8, 4, 2, 1, or 0.5 hours after the other component.

In sequential administration, the extract may be administered once, or any number of times and in various doses before and/or after administration of the therapeutic compound. Sequential administration may be combined with simultaneous or sequential administration.

### Kit

Another aspect of the present invention is a kit comprising a container in which an extract of *Calotropis procera* is present, and a container in which a therapeutic compound is present. An extract according to the invention and therapeutic compound according to the invention are described above.

By mentioning a kit comprising a container in which an extract of *Calotropis procera* is present, and a container in which a therapeutic compound, it is to be understood that the kit may contain a single dose, or a plurality of doses, reflected in two single containers, two single containers each capable of holding a plurality of doses or a plurality of single containers each capable of holding a single dose. It is to be understood that the kit may comprise more than one therapeutic compound, each therapeutic compound in a separate container, or a mixture of therapeutic compounds in a single container.

The containers may be any known in the art of medicines. They may be vials, blister packs, syringes, resealable bottles, bottles with dispensing means, aspirators, dropping bottles, patches, applicators, suppositories.

The separate containers enable to extract and therapeutic compound to be administered simultaneously, separately, or sequentially.

### Uses

Due to the favorable anti-tumor properties of at least one of the extracts according to the present invention, said extracts are particularly useful in the treatment of individuals suffering from cancer. Due to its low level of toxicity and its minimal side effects, use of one said extract in a pharmaceutical composition for the treatment of cancer will involve minimal side effects. As a consequence, a said the extract according to the invention may be used during a longer period of time during the treatment of cancer.

In particular, in a preferred embodiment, the invention relates to the use of a composition according to claim 1 for preparing a medicament for treating cancer, wherein the cancer is selected from the group comprising, but not limited to breast cancer, lymphoma, sarcoma, pancreatic cancer, melanoma, colorectal cancer, glioma, non small cell lung cancer, small cell lung cancer, skin cancer, bone cancer, ovarian cancer, CNS cancer, renal cancer, bladder cancer, head and neck cancer, prostate cancer, liver cancer and hematological cancer.

For these purposes, the pharmaceutical composition of the present invention may be administered orally, parenterally, i.e. including subcutaneous injections, intravenous, intramuscular, intrasternal injection or infusion techniques, by inhalation spray, or rectally, in dosage unit formulations containing conventional non-toxic pharmaceutically acceptable carriers, adjuvants and vehicles.

In accordance with the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

Essentially, the primary modes of treatment of solid tumor cancers comprise surgery, radiation therapy and chemotherapy, separately and in combination. The extracts according to the invention are suitable for use in combination with these medicinal techniques. The extracts according to the invention may be useful in increasing the sensitivity of tumor cells to radiation in radiotherapy and also in potentiating or enhancing damage to tumors by chemotherapeutic agents. The extracts according to the invention may also be useful for sensitizing multidrug-resistant tumor cells. The extracts according to the invention are useful therapeutic compounds for administration in conjunction with other DNA-damaging therapeutic compounds to potentiate their effect.

It will be understood, that the pharmaceutical composition according to the invention can be administered to humans in specific dose levels and at specific frequency of dosage which may be varied for any particular patient and which will depend upon a variety of factors including the age, body weight, general health, sex, diet, mode and time of administration, rate of excretion, drug combination, the severity of the particular condition of the patient undergoing therapy.

The following examples are meant to illustrate the present invention. These examples are not to be considered as limiting the scope of the invention. A first example illustrates the extraction of extracts according to the invention form the plant *Calotropis procera.* Examples 2 to 4 relate to the *in vitro* anti-tumor characterization of an extract according to the invention. Examples 5 to 7 describe the *in vivo* anti-tumor characterization of an extract according to the invention. Example 8 illustrates the anti-poisonous effects of extracts according to the invention.

### EXAMPLES

### Example 1 Extraction process of extracts according to the invention from the plant Calotropis procera

An extract (Extract A) according to the invention was isolated form the roots of *Calotropis procera* plant *(Asclepiadiaceae* family) by methanol. The said extract is further referred as Extract A *(to adapt).* About 10 grams of plant was put in an erlenmeyer with 150 ml of methanol. The suspension was shaken magnetically for 12 hours and then filtered on a glass frit. The remaining solid was extracted a second time by methanol for 2 hours. Both filtrates were combined and evaporated under vacuum by using rotavapor. The residue constituted the methanolic extract is referred hereafter as Extract A.

Analysis of Extract A revealed the presence of several types of compounds. A particular group of compounds identified in said Extract A comprises cardenolides such as asclepin, calactin, vorusharin, calotropin, calotropagenin, uzarigenin, usharin and usharidin. In addition, some other compounds, present in Extract A comprise, but are not limited to, 3-O-ACETYL-CALOTROPIN, ALPHA-AMYRIN, ALPHA-AMYRIN-BENZOATE, ALPHA-CALOTROPEOL, ALPHA-LACTUCEROL, ALPHA-LACTUCERYL-ACETATE, ALPHA-LACTUCERYL-ISOVALERATE, ARABINOSE, BENZOYLISOLINEOLONE, BENZOYLLINEOLONE, BETA-AMYRIN, BETA-AMYRIN-BENZOATE, BETA-CALOTROPEOL, BETA-SITOSTEROL, CAOUTCHOUC, COROGLAUCOGENIN; COROTOXIGENIN; D-GLUCOSAMINE, FRUGOSIDE, GIGANTEOL, GIGANTIN, GLUCOSE, HISTAMINE, ISOGIGANTEOL, ISOLACTUCEROL, ISOLINEOLONE, LAURANE LINEOLONE, LINOLEIC-ACID, LINOLENIC-ACID, MELISSYL-ALCOHOL, MUDARINE, OLEIC-ACID, PALMITIC-ACID, PROCEROSIDE, PSEUDOCALOTROPAGENIN, RHAMNOSE STIGMASTEROL, SYRIOGENIN TARAXASTEROL, TARAXASTEROL-BENZOATE and TRYPSIN.

Other extracts according to the invention, where isolated by a method, comprising the steps of:
a) Grinding the starting material of leaf blades, stems, barks and roots of *Calotropis procera* to give a fine powder of the plant,
b) Extracting the powder of step a) with dichloromethane for 24 hours using a soxhlet extractor,
c) decanting the dichloromethane of step b), and evaporating the filtrate (after filtration) to form a gum,
d) extracting the residue of step c) with methanol for 24 hours using a soxhlet extractor,
e) decanting the methanol of step d), evaporating the filtrate (after filtration) to form a gum,
f) subjecting the gum of step e) to column chromatography using flash silica gel and dichloromethane-methanol as solvent,
g) collecting a first fraction,
h) applying the concentrated fraction of step g) to column chromatography using flash silica gel and hexane-acetone as solvent to give two fractions,
i) washing the column after step h) with methanol to give a third fraction.

The extract isolated at step c) is referred hereafter as Extract B. The extract isolated at step g) is referred hereafter as Extract C. The extract isolated at step i) is referred hereafter as extract D.

### Example 2 Effect of an extract according to the invention on overall cell growth of a cell line

This example illustrates the anti-tumor activities of Extract A according to the invention on different types of cancer.

In order to characterize the *in vitro* activities of the Extract A, MTT tests were carried out. The MTT test, which is a well known test in the art, is an indirect technique that rapidly measures, i.e. within 5 days, the effect of a given product on the overall growth of a cell line. This test measures the number of metabolically active living cells that are able to transform the MTT product (3-(4,5-dimethylthiazol-2-yl)-2,5 diphenyl tetrazolium bromide), having a yellowish color, to the blue product formazan by mitochondrial reduction only performed by living cells. The amount of formazan obtained at the end of the experiment is measured with a spectrophotometer and is directly proportional to the number of living cells.

Forty-eight human cancer cell lines, described in Table 2, were tested in the presence of Extract A. These cell lines covered ten histological types, being pancreatic cancer, sarcoma, breast cancer, melanoma, colon cancer, glioma, lung cancer, bladder cancer, prostate cancer and head and neck cancer. The cells were allowed to grow in flat bottomed 96-well micro-wells with 100 µl of cell suspension per well and between 3,000 and 5,000 cells/well depending on cell type. Each cell line was seeded in its own cell culture medium as indicated in Table 2.

**TABLE 2 Human cancer cell lines and corresponding cell culture medium used for the MTT experiments**

| **Cell lines** | **ATCC code** | **Tissue** | **Medium** |
|---|---|---|---|
| **BxPC-3** | CRL-1687 | Pancreatic | RPMI 10% serum |
| **MiaPACA-2** | CRL-1420 | Pancreatic | DMEM glucose 10% |
| **PANC-1** | CRL-1469 | Pancreatic | OPTIMEM 5% |
| **CAPAN-1** | HTB-79 | Pancreatic | RPMI 10% serum |
| **CFPAC-1** | CRL-1918 | Pancreatic | Iscove's 10% serum |
| **Hs766T** | HTB-134 | Pancreatic | OPTIMEM 5% |
| **SU.86.86** | CRL-1837 | Pancreatic | RPMI 10% serum |
| **SK-LMS-1** | HTB-88 | Sarcoma | MEM 10% serum |
| **SK-UT-1B** | HTB-115 | Sarcoma | MEM 10% serum AA |
| **HT-1080** | CCL-121 | Sarcoma | MEM 5% serum |
| **Hs729** | HTB-153 | Sarcoma | MEM 10% serum AA |
| **MES-SA** | CRL-1976 | Sarcoma | MEM 5% serum |
| **RD** | CCL-136 | Sarcoma | MEM 5% serum |
| **A204** | HTB-82 | Sarcoma | MEM 5% serum |
| **MCF-7** | HTB-22 | Breast | MEM 5% serum |
| **T-47D** | HTB-133 | Breast | MEM 5% serum |
| **MDA-MB-231** | HTB-26 | Breast | DMEM Nut mix 10% serum |
| **ZR-75-1** | CRL-1500 | Breast | MEM 5% serum |
| **Hs578T** | HTB-126 | Breast | MEM 5% serum |
| **SK-MEL-28** | HTB-72 | Melanoma | RPMI 10% serum |
| **HT-144** | HTB-63 | Melanoma | MEM 10% serum AA |
| **C-32** | CRL-1585 | Melanoma | MEM 5% serum |
| **Malme-3M** | HTB-64 | Melanoma | MEM 5% serum |
| **G-361** | CRL-1424 | Melanoma | OPTIMEM 5% serum |
| **HCT-15** | CCL-225 | Colon | MEM 5% serum |
| **LoVo** | CCL-229 | Colon | MEM 5% serum |
| **DLD-1** | CCL-221 | Colon | MEM 10% serum AA |
| **Ls-174T** | CL-188 | Colon | MEM 10% serum AA |
| **HT29** | HTB-38 | Colon | MEM 10% serum AA |
| **WiDR** | CCL-218 | Colon | MEM 10% serum AA |
| **SW948** | CCL-237 | Colon | OPTMEM 5 % serum |
| **A172** | CRL-1620 | Glioma | MEM 5% serum |
| **H4** | HTB-148 | Glioma | MEM 5% serum |
| **Hs683** | HTB-138 | Glioma | MEM 5% serum |
| **SW1088** | HTB-12 | Glioma | MEM 5% serum |
| **U-118 MG** | HTB-15 | Glioma | MEM 5% serum |
| **U-87 MG** | HTB-14 | Glioma | MEM 5% serum |
| **U-373 MG** | HTB-17 | Glioma | MEM 5% serum |
| **A427** | HTB-53 | Lung | MEM 5% serum |
| **A549** | CCL-185 | Lung | MEM 5% serum |
| **J82** | HTB-1 | Bladder | MEM 5% serum |
| **T24** | HTB-4 | Bladder | MEM 5% serum |
| **PC3** | CRL-1435 | Prostate | MEM 5% serum |
| **Detroit** | CCL-138 | Head and neck | MEM 10% serum AA |
| **RPMI** | CCL-30 | Head and neck | MEM 10% serum AA |
| **FaDu** | HTB-43 | Head and neck | MEM 10% serum AA |
| **SCC9** | CRL-1629 | Head and neck | MEM 10% serum AA |
| **SCC25** | CRL-1628 | Head and neck | MEM 10% serum AA |

| | | | |
|---|---|---|---|
| Where AA means "Amino Acids" | | | |

After a 24-hour incubation period at 37°C the culture medium was replaced by 100 µl of fresh medium in which Extract A was dissolved at the different concentrations of 0.01 µg/ml, 0.05 µg/ml, 0.1 µg/ml, 0.5 µg/ml, 1 µg/ml, 5 µg/ml, 10 µg/ml, 50 µg/ml and 100 µg/ml. Each experimental condition was carried out in sextuplicate.

After 72 hours of incubation at 37°C with the drug, i.e. experimental conditions or without the drug, *i.e.* control, the medium was replaced by 100 µl MTT at the concentration of 1 mg/ml dissolved in RPMI. The micro-wells were then incubated for 3 hours at 37° C and centrifuged at 400g for 10 minutes. The MTT was removed and the formed formazan crystals were dissolved in 100 µl DMSO. The micro-wells were shaken for 5 minutes and read on a spectrophotometer at 2 wavelengths at 570 nm corresponding to the maximum formazan absorbance wavelength, and at 630 nm, which is the background noise wavelength.

For each experimental condition, the mean OD associated with the standard error of the mean (SEM) for each condition, i.e. 6 wells, was calculated. The percentage of remaining living cells was calculated in comparison with control. Results of these experiments are represented in figures 1 to 11.

Figure 1 represents the overall results for the ten histological types. As indicated Extract A exerted an anti-tumor effect on all histological types tested. The human tumor cell lines issued from bladder presented a sensitivity to Extract A which was weaker than the remaining nine histological types. The concentration at which the plant extract killed 50% of the cells population, the so-called IC₅₀ value, was determined. Said IC₅₀ value comprised between 0.5 µg/ml and 1 µg/ml for the cell lines issued from the breast and the pancreas cell lines. A more important anti-tumor effect with a IC₅₀ value of 0.5-0.1 µg/ml was obtained for sarcoma, melanoma, glioma, lung, head and neck and colorectal, cancer cell lines. The PC3 prostate cancer cells exhibited a marked sensitivity, with an IC₅₀ value in range of 0.01 to 0.05 µg/ml.

As illustrated on figure 1, the mean IC₅₀ value of the 5 breast cancer cell lines ranged between 0.5 and 1 µg/ml. The overall growth of the 5 individual breast cancer cell lines, T-47D, MDA-MB-231, ZR-75-1, MCF-7 and Hs578T, is further illustrated in figure 2. The cell lines T-47D, MDA-MB-231, ZR-75-1 and Hs578T exhibited similar sensitivities to Extract A, while MCF-7 cells had their overall growth reduced more rapidly than the remaining four breast cancer models.

As illustrated on figure 1, the mean IC₅₀ value of the 7 sarcoma cancer cell lines (see Table 2) ranged between 0.5 and 0.1 µg/ml. The overall growth of the individual cell lines is further illustrated in figure 3. Extract A induced the most important anti-tumor effect on the A204 cell line. In fact, the IC₅₀ value appeared to be between 0.1 and 0.05 µg/ml. The Hs729 cell line was considered to the least sensitive of the 7 sarcoma cell lines. It nevertheless showed IC₅₀ values for Extract A ranging between 1 and 5 µg/ml.

As illustrated in figure 1, the mean IC₅₀ value of the 7 pancreatic cancer cell lines (see Table 2) ranger between 0.5 and 0.1 µg/ml. Similar overall growths were observed for 6 of the 7 pancreatic cancer cell lines (Figure 4). Of these 7 cell lines, Panc-1 seemed to be the most sensitive to Extract A and Hs766T the least. The IC₅₀ values of these 2 lines ranged between 0.1 and 0.5 µg/ml, and between 1 and 5 µg/ml respectively.

As illustrated on figure 1, the mean IC₅₀ value of the 5 melanoma cancer cell lines (see Table 2) ranged between 0.5 and 0.1 µg/ml. The extract according to the invention reduced the overall growth of all the melanoma cell lines by more than 60% at concentrations equal to or higher than 5 µg/ml (Figure 5). Malme-3M and G-361 were the most sensitive of the 5 melanoma cell lines. They exhibited a similar overall growth, with the IC₅₀ value ranging between 0.1 and 0.5 µg/ml.

As illustrated on figure 1, the mean IC₅₀ value of the 7 colon cancer cell lines (see Table 2) ranged between 0.5 and 0.1 µg/ml. Of the different colorectal cancer cell lines tested (Figure 6) the LoVo line had an IC₅₀ value of around 0.1 µg/ml and was considered to be the most sensitive colorectal line. The SW948 tumor cell line was considered to be the least sensitive, with a IC₅₀ value ranging between 0.5 and 1 µg/ml.

As illustrated in Figure 1, the mean IC₅₀ value of the 7 human glioma cancer cell lines (see Table 2) ranged between 0.5 and 0.1 µg/ml. Hs683 was the most sensitive cell line to extract (Figure 7). Its IC₅₀ value was near 0.05 µg/ml. In contrast, the growth of A172 cells was not affected by Extract A at concentrations from 0.01 to 10 µg/ml. The IC₅₀ value ranged between 10 and 50 µg/ml.

As illustrated on figure 1, the mean IC₅₀ value of the 2 human non-small-cell-lung cancer cell lines (NSCLC) ranged between 0.1 and 0.5 µg/ml. Figure 8 shows that both lines were sensitive to Extract A.

As illustrated on figure 1, the mean IC₅₀ value of the 2 human bladder cancer cell lines (see Table 2) ranged between 50 and 100 µg/ml. The 2 lines exhibited marked differences in term of sensitivity to Extract A (Figure 9). In fact, at 5 µg/ml of Extract A reduced the overall growth of the T24 cell line by more than 80% while the overall growth of the J82 cell line was only decreased by 32% at 100 µg/ml. The J82 cell line seemed very weakly sensitive to Extract A.

The overall growth of 1 prostate cancer cell line (the PC3 cell line) was evaluated by MTT assay when the cells had been treated with Extract A. Extract A induced an inhibition of overall growth of about 90% at between 100 µg/ml and 0.5 µg/ml. The IC₅₀ value was around 0.1 µg/ml (Figure 10).

As illustrated on figure 1, the mean IC₅₀ value of the 5 head and neck cancer cell lines ranged between 0.1 and 0.5 µg/ml. Similar overall growths were observed for 4 of the 5 head and neck cancer cell lines (Figure 11). Of these 5 cell lines, SCC9 seemed to be the least sensitive. It nevertheless showed IC₅₀ values for Extract A of around 5 µg/ml.

Summarized, Extracts A according to the invention exerts a dramatic anti-tumor effect on forty-six of the forty-eight human cancer cell lines assayed in the experiments described above. These anti-tumor effects correspond to marked decreases in the overall growth of these human cancer models representing a very broad panel of histological types.

### Example 3 Effect of an extract according to the invention on cell kinetics

This example illustrates the cytostatic effect of Extract A according to the invention. According to the experiments performed by means of the MTT colorimetric assay described in example 2, it is clear that Extract A dramatically decreases the overall growth of most of the fourty-eight human cancer cell lines submitted to the MTT assay. In the following examples it was investigated whether this extract induced decrease in overall growth corresponds either to modifications occurring at the levels of the cell cycle kinetics (example 3), or the induction of apoptosis (see example 4), or of both.

The cell cycle is in general divided in several phases comprising a G0/G1, S and G2/M phase. Modifications taking place around the proteins controlling cell proliferation and/or cell death may constitute one target of the active compounds, present in Extract A. Modifications to cell cycle kinetics can be investigated by means of flow cytometry using different fluorophores f.e. propidium iodide, orange acridine and ethidium bromide etc. Flow cytometry enables each cancer cell (running to several thousands) to be located into the cell cycle.

Changes in the DNA histogram pattern are thus used to characterize the mechanism of action of various therapeutic compounds. The data resulting from the flow cytometry analysis is processed graphically or mathematically in order to derive meaningful estimates of the G1, the S and the G2/M compartments. Most mathematical processing is based on certain models and assumptions.

Cell lines were seeded in flasks (25 cm² area) containing 7 ml of culture medium. After 48 hours incubation at 37°C the cell culture medium was replaced by fresh medium in which Extract A had been dissolved at concentrations which kill 50%, (IC₅₀), 30% (=IC₃₀) and 10% (IC₁₀) of the cell population. After 24 or 72 hours of treatment the cells were harvested in suspension, washed in Phosphate Buffer Saline (PBS) at 4°C and permeabilized with 70 % ethanol (at 4°C) overnight at -20°C. The cells were then washed with PBS and incubated with propidium iodide solution (80 µg/ml) for 30 minutes at 37°C and afterwards kept at 4°C overnight. Ribonuclease A (3% V/V) was added to induce a double-stranded DNA break. The portrait of the cell cycle was established for each sample. A specific software program incorporated into the flow cytometer was used to define precisely the percentage of cells in the different cell cycle phases. Each cell cycle phase was reported in terms of peak surface and calculated as a percentage. The surface of the entire cell cycle was 100 %. Each experiment was carried out 3 times. The mean percentage of each different phase and the standard error of the related mean was calculated. Each cell cycle phase of a given condition was compared with the same cell cycle phase of control cells, i.e non-treated cells.

Extract A is highly effective against human tumor cell lines. The concentrations used in our flow cytometry experiments were chosen in accordance with the MTT results (example 2). The five human cancer cell lines Hs683 (glioma), J82 (bladder) A172 (glioma), RPMI (head-and-neck) and HCT-15 (colon) were tested and doses that killed 10, 30 and 50 percent of the cells were determined (Table 3) in order to investigate whether Extract A promoted an accumulation in one of the cell cycle phases when the cultures were treated for 24 or 72 hours with increasing concentrations of Extract A.

**TABLE 3 Doses of Extract A according to the invention killing 10, 30 and 50 % of the cells in five cell lines**

| **Cell lines** | **IC₅₀** | **IC₃₀** | **IC₁₀** |
|---|---|---|---|
| HCT-15 | 0.25 µg/ml | 0.1 µg/ml | 0.05 µg/ml |
| RPMI | 0.25 µg/ml | 0.1 µg/ml | 0.05 µg/ml |
| A172 | 25 µg/ml | --- | 10 µg/ml |
| J82 | --- | 50 µg/ml | 10 µg/ml |
| Hs683 | 0.05 µg/ml | --- | 0.025 µg/ml |

The HCT-15 cell cycle analyses (Figure 12) showed that the distribution of the cell population was similar to the control after 24 hours of treatment independent of the concentrations tested. On the other hand, a prominent S-population appeared in the cells treated with 0.25 µg/ml extract for 72 hours. The S fraction represented 21% of the cells in control and reached 59% upon treatment with Extract A at 0.25 µg/ml. The increase in the S fraction was accompanied by a loss of cells in the G0/G1 phase. The G0/G1 population underwent a marked decrease from 71% to 29%.

The analyses of the RPMI cell cycle (Figure 13) showed that the distribution of the cell population treated with Extract A at 0,05 µg/ml was similar to the control after 24 hours of treatment. A weak increase in phase S was observed at 0,1 and 0,25 µg/ml. In contrast, an large increase in the S-population occurred when the RPMI cell line was incubated for 72 hours with Extract A at the three concentrations tested. There were about 71%, 69% and 62% of cells in the S-phase when Extract A was assayed at 0.05 µg/ml, 0.1 µg/ml and 0.25 µg/ml, respectively. Concomitantly, the percentage of cells in the G0/G1 phase decreased markedly and the G2/M phase increased slightly, reaching 29% of the cell population.

The A172 cell line was treated with Extract A at 10 and 25 µg/ml, which corresponds to the IC₁₀ and IC₅₀. The results (Figure 14) showed that whatever the concentrations tested Extract A induced an increase in the G0/G1 phase after 24 hours of treatment. Thirty-nine percent of the cells were in the G0/G1 phase in control while the cells treated with Extract A reached 60%. Concomitantly with the accumulation in the G0/G1 phase, we observed a slight accumulation in the G2/M phase at the greatest concentration tested. The effect observed after 24 hours of treatment disappeared after 72 hours. Indeed, no significant change was observed in the different cell cycle phases.

The J82 cell line is hardly sensitive to the extract (cfr. MTT assay). The concentrations chosen for flow cytometry corresponded to about IC₁₀ and IC₃₀ (Figure 15). After 24 hours of treatment a slight accumulation in G0/G1 phase was obtained whatever the concentration tested. On the other hand, 30 and 47% respectively of the cell population was in the S phase, when the cell were treated for 72 hours with Extract A at 50 and 100 µg/ml while the control condition reached only 18%. At 100 µg/ml, an accumulation in G2/M phase could also be observed.

After 24 hours of treatment with Extract A at 0.05 µg/ml the Hs683 cells had accumulated in the G0/G1 phase and attained 70% as compared to control (58%) (Figure 16). We observed concomitantly a decrease in the percentage of cells in the S phase. In the same way, at the both concentrations most of the cells were in the G0/G1 phase after 72 hours of treatment. Indeed, more than 60% of the cells were in this phase while the cells in this phase in control represented 46% of cell population.

In conclusion, Extract A principally induces an accumulation in the G0/G1 phase and under such circumstances this indicates that Extract A has a cytostatic effect.

### Example 4 Pro-apoptotic effect of an extract according to the invention

This example illustrates the pro-apoptotic effect of Extract A according to the invention.

Cell death may occur in two different ways, either accidentally or as genetically programmed. Accidental cell death, also referred to as necrosis essentially occurs as the results of e.g. physical or biological aggression. Apoptosis refers to the form of cell death which is genetically programmed cell death and which occurs under normal physiological conditions. After the induction of apoptosis, a cascade of events is induced in the cell, which comprises the activation of cell death receptors, the activation of a serie of cytosolic proteases, the formation of apoptotic bodies, the fragmentation of the DNA.

The effect of Extract A on the apoptosis pathway was investigated on the four human cancer cell lines: Hs683 (glioma), J82 (bladder), A172 (glioma) and HCT-15 (colon).

Cells were seeded in flasks (25 cm² area) containing 7 ml of culture medium. After a 48-hours incubation at 37°C the cell culture medium was replaced by fresh medium in which Extract A according to the invention was dissolved at different concentrations that killed 50% and 30% of cell population. After 24 or 72 hours of treatment cells were harvested in suspension and counted. 250.000 cells were centrifuged for 10 minutes at 1700 rpm at 4°C. The pellet was washed with PBS at 4°C and incubated with annexin V-FITC and propidium iodide solution for 15 minutes at 4°C in the dark. For each sample, data from approximately 10,000 cells were recorded on logarithmic scale. Software incorporated into the flow cytometer was used to define precisely the percentage of cells in apoptotic and/or necrotic pathway, and the normal cells. Each experiment was realized two times. The mean of percentage of both the apoptotic and the necrotic way and the standard error of the related mean was calculated. Each condition was compared to the control, being non-treated cells.

Four-fold increase of apoptotic HCT-15 cells was observed when cells were treated by Extract A at 0.25 µg/ml for 24 hours as compared to the control cells (Figure 17). The A172 cell line seemed to be engaged in an apoptosis and necrosis pathway when the cells were treated for 24 hours by Extract A at 25 µg/ml (Figure 18). This tendency was maintained after 72 hours of treatment. The treatment by Extract A induced the apoptosis of J82 cells in a concentration-dependent manner, which is particularly clear 72 hours after treatment (Figure 19). Also an increase in the percentage of necrotic cells is observed at both concentrations at 72 hours. A 2.5 fold increase in the percentage of Hs683 apoptotic cells was obtained after 24 hours of treatment with Extract A at 0.025 µg/ml (Figure 20). After 72 hours of treatment the percentage of apoptotic cells increased in concentration-dependent manner.

In conclusion, Extract A principally induces an increase in the percentage of apoptotic cells. An effect on the necrosis pathway was obtained under certain conditions.

### Example 5 Maximum tolerated dose of an extract according to the invention

In the present example, the MTD index was determined for Extract A according to the invention. The Maximum Tolerated Dose (MTD) of a given drug is defined as the maximum amount of a drug which can be administered acutely, i.e. in one intraperitoneal, intravenous, subcutaneous or *per os* single dose, to healthy animals, i.e. animals not grafted with tumors.

The experimental conditions to determine the MTD index of Extract A were the following. The survival times of mice, which are not grafted with a tumor, were recorded up to 14 days post-injection. Six different doses of Extract A were used for the determination of the MTD index. The highest dose administered to tumor-bearing mice was 160 mg/kg. Other doses comprised 5mg/kg, 10mg/kg, 20mg/kg, 40mg/kg and 80mg/kg. Each experimental group was composed of two mice for the determination of the MTD index. Table 4 below shows the data obtained for the MTD index using Extract A.

**TABLE 4 MTD index determination for Extract A according to the invention**

| **Administered doses of extract (mg/kg)** | **Day 1 post administration** | **Day 14 post administration** |
|---|---|---|
| 1 x 5 | -- | -- |
| 1 x 10 | -- | -- |
| 1 x 20 | -- | -- |
| 1 x 40 | X | |
| 1 x 80 | XX | |
| 1 x 160 | XX | |

| | | |
|---|---|---|
| where x means one dead mouse and - means all the animals remained alive | | |

According to the definition given above, the MTD index for Extract A is 20 mg/kg for single administration in mice. Thus, the Maximum Tolerated Dose (MTD) index of Extract A, referring to the maximum amount of Extract A, which can be administered acutely to healthy animals, is 20mg/kg extract. This value indicates that Extract A has a broad therapeutic window.

### Example 6 In vivo effects of an extract according to the invention evaluated on three cancer models

This example illustrates the *in vivo* effects of the Extract A according to the invention on three different cancer models.

The *in vivo* effects of Extract A were studied on mice grafted with different types of tumors, including a lymphoma cancer, a melanoma cancer and a breast cancer. The *in vivo* effects were evaluated with three types of parameters:
- the *cumulative toxicity,* which is evaluated by recording the weights of the tumor-bearing mice during treatment
- the *actual anti-tumor effect* exerted at tumor growth level, which is evaluated by measuring tumor size three times a week by means of a caliper. The actual tumor growth is expressed as an area (mm²) by multiplying the two largest perpendicular diameters.
- the *survival gain* for the mice treated, which is calculated by means of the T/C index. This index is the ratio between the median survival time of the group of treated mice (T) and that of the control group (C). The extract is considered to be active if the T/C value is above 130 % (P<0.05), very active for a value higher than 150% (P<0.01) and toxic for a value lower than 70%.

### In vivo effects of Extract A on a lymphoma cancer model

Extract A was evaluated on the aggressive P388 lymphoma cancer model. In a first experiment three doses, 10mg/kg, 5mg/kg and 2.5mg/kg were compared to control. The mice were inoculated subcutaneously with 10⁶ P388 cells at day D0 and treated nine times during the three following weeks at days D5, D7, D9, D12, D14, D16, D19, D21 and D23 post-graft. Each experimental group contained nine mice.

Figures 21A illustrates that Extract A did not influence the body weight of the mice. Figure 21 B shows that Extract A significantly induced a decrease in P388 lymphoma growth. The T/C index values for the 7x10 mg/kg administration schedule gave a T/C index of 111 %, the T/C index values for the 6 x 5 mg/kg schedule gave a T/C index of 100 % and the T/C index values for the 8 X 2.5 mg/kg schedule gave a T/C index of 121 %. In conclusion, these results indicated that administration of Extract A decreased the growth of the P388 lymphoma cancer but did not significantly prolong the survival of the P388 lymphoma-bearing mice at the tested concentrations.

In a second set of experiments, the number of administrations was increased from nine to sixteen, accompanied by a concomitant decrease in the dose per single administration. The subcutaneously-administered doses were 2.5mg/kg, 1.25 mg/kg and 0.63mg/kg. Extract A was administered daily, five days a week, for five consecutive weeks (5 x 5 = 25).

Figure 22B shows that these active Extract A doses of 0.63 mg/kg and 1.25 mg/kg had negligible toxic effects since the P388 lymphoma-bearing mice lost no weight during treatment. Figure 22A shows that Extract A markedly decreased P388 lymphoma growth at doses of both 0.63 mg/kg and 1.25 mg/kg. In addition, the T/C index values for the 15 x 2.5 mg/kg administration schedule gave a T/C index of 137 %, that the T/C index values for the 15 x 1.25 mg/kg schedule gave a T/C index of 137 % and that the T/C index values for the 13 × 0.63mg/kg schedule gave a T/C index of 116%. Thus, the 15 administrations of 2.5 mg/kg Extract A and the 15 administrations of 1.25 mg/kg Extract A significantly increased the survival of these P388 lymphoma-bearing mice by 37%. Consequently, the treatments at doses of 2.5 mg/kg and 1.25 mg/kg with Extract A prolonged the survival of P388 lymphoma-bearing mice.

In conclusion, Extract A according to the invention exerts significant anti-tumor effects on the aggressive P388 lymphoma cancer model without any loss of body weight in the animals concerned. Also, unexpectedly, the Extract A according to the invention exerts higher anti-tumor activity when assayed chronically at low doses, i.e. around 1.25 to 0.63mg/kg, than at high doses, i.e. around 10mg/kg to 5 mg/kg.

### In vivo effects of Extract A on a melanoma cancer model

Extract A was evaluated on the aggressive B16 melanoma cancer model. Extract A was subcutaneously administered to mice at doses of 2.5mg/kg, 1.25 mg/kg and 0.63mg/kg. Extract A was administered daily, five days a week, for five consecutive weeks.

Figure 23A shows that the administered doses had negligible toxic effects since the B16-melanoma-bearing mice lost no body weight during the treatment. Figure 23B shows that Extract A administrations dispensed at 2.5 or 1.25 mg/kg significantly decreased the growth of the B16 melanoma. In addition, the T/C index values for the 20 x 2.5 mg/kg administration schedule gave a T/C index of 117 %, the T/C index values for the 20 x 1.25 mg/kg schedule gave a T/C index of 117 % and the T/C index values for the 25 × 0.63mg/kg schedule gave a T/C index of 140%. Thus, Extract A decreased the growth of the B16 melanoma and significantly prolonged the survival of the B16 melanoma-bearing mice, especially when Extract A was administered 25 times at 0.63 mg/kg when the level of significance is reached.

In a second set of experiments, Extract A was administered orally (per os).
Figure 24A shows that the administered doses had negligible toxic effects since the B16-melanoma-bearing mice lost no body weight during the treatment. Figure 24B shows that the Extract A administrations *per os* at 2.5 or 1.25 mg/kg significantly decreased the growth of the B16 melanoma up to 25 days post-graft. In addition, the T/C index values for the 19 x 2.5 mg/kg administration schedule gave a T/C index of 114%, the T/C index values for the 15 × 1.25 mg/kg schedule gave a T/C index of 100% and the T/C index values for the16 x 0.63 mg/kg schedule gave a T/C index of 104%. Thus, Extract A induced a decrease in the growth of the B16 melanoma tumor and slightly prolonged the survival of the B16 melanoma-bearing mice at 2.5mg/kg.

In conclusion the two sets of experiments provide evidence that Extract A according to the invention exerts significant anti-tumor effects on the B16 melanoma model independent of the mode of administration.

### In vivo effects of Extract A on a breast cancer model

Extract A was evaluated on the MXT-HI breast cancer model, i.e. a hormone insensitive variant of breast cancer. The MXT-HI model corresponds to an undifferentiated carcinoma with dramatic metastatic processes towards the liver. It therefore corresponds to the late clinical stages of human breast cancer. The MXT-HI represents a very aggressive biological tumor model.

The MXT-HI breast cancer is induced in mice by subcutaneously injecting MXT tumor fragments into the flanks of B6D2F1 mice. Without treatment, the inoculated mice die between the fourth and seventh week after the inoculation. Extract A was assayed at 10 mg/kg, 5 mg/kg and 2,5 mg/kg with nine administrations, i.e. three times a week for three consecutive weeks.
Figure 25A indicates that the various Extract A treatment schedules used in the present experiments induced essentially no major toxic-side effects since the MXT-HI-bearing tumor mice did not lose significant weight. Figure 25B shows that nine administrations of 2.5 mg/kg significantly decreased MXT-HI tumor growth. The T/C index values for the 9 x 10 mg/kg administration schedule gave a T/C index of 103%, the T/C index values for the 9 x 5 mg/kg schedule gave a T/C index of 103% and the T/C index values for the 9 x 2.5 mg/kg schedule gave a T/C index of 119%. Thus at the dose of 2.5 mg/kg, Extract A prolonged the survival of the MXT-HI-bearing mice.

In Figure 26 the number of the MXT-HI breast cancer bearing mice, which died during the experiment in the control (untreated) and test groups (treated with extract), is shown. A Kaplan-Meier statistical analysis was used and represents the death rate of the nine mice in each group. The statistic value underlines the general fit of the test group in comparison with the general fit of the control group with regard to survival.

In conclusion the experiments described above provide evidence that Extract A exerts significant anti-tumor effects on the MXT-HI breast cancer tumor.

### Conclusions on example 6

The Extract A according to the invention has a significant anti-tumor effect on the tested cancer models. These models represent a broad panel of histological tumor types, including carcinomas, lymphomas and melanomas. These models are clinically relevant because they mimic specific clinical stages of human cancers. Apart from this, they are also biologically aggressive and invasive because two of them metastasize dramatically to the liver.

While having a very significant anti-tumor effect, Extract A according to the invention exhibits negligible toxic effects since the tumor-bearing mice did not lose body weight during the treatments. The semi-purified extract can therefore include an "antidote" in addition to the anti-tumor compound responsible for all the anti-tumor activities reported here.

Surprisingly, Extract A induces significantly higher anti-tumor activities when assayed chronically at low doses i.e. around 1.25 to 0.63mg/kg, than at high doses, i.e. around 10mg/kg to 5 mg/kg.

### Example 7 In vivo toxicological effects of an extract according to the invention

This example illustrates the toxicological effects of Extract A according to the invention, in particular the *in vivo* hematotoxicity and general toxicity.

### Hematotoxicity

Hematotoxicity is evaluated by establishing hematological profiles for each animal species. Particular attention was paid to the numbers of blood platelets, red cells and leukocytes. The effect of Extract A was evaluated at two doses, i.e. 5 mg/kg and 1.25 mg/kg, by the intra-peritoneal administration to mice. The administration schedule was five administrations a week for five consecutive weeks resulting in a total injection number of twenty-five. The animals were sacrificed three days after the last injection. There were ten mice per group.

As compared to control (Figure 27), no statistically significant changes were observed with respect to hematological parameters after treatment with Extract A. No significant changes were observed on the mean cell volume of red blood cells, the mean corpuscular hemoglobin, the mean corpuscular hemoglobin concentration and the platelets after the treatments.

### General toxicity

The general toxicity of Extract A was histologically tested on mice by means of conventional histopathological analyses of hematoxilin-eosin-stained histological slides obtained from different types of organs and tissues. The effect of Extract A was evaluated at 5 mg/kg and at 1.25 mg/kg by intra-peritoneal administration. The administration schedule was five administrations a week for five consecutive weeks resulting in a total injection number of twenty-five. The animals were sacrificed three days after the last injection. The brain, the heart, the liver, the pancreas, the stomach, the intestines and the ovaries were collected. There were ten mice per group.

Examination of the collected organs showed that control (untreated) group did not show any particular modification in the organs. Also in treated mice the brain, the heart, the liver, the pancreas, the stomach, the intestines and the ovaries were not affected by Extract A treatments at doses of 5mg/kg or 1.25 mg/kg, indicating that Extract A does not have a general toxic effect.

### Example 8 Anti-poisonous effects of extracts according to the invention

This example illustrates the anti-poisonous effect of the extracts according to the invention. Female mice of 4 to 5 weeks of age were injected with two or four times the maximal tolerable dose of two well-known and clinically used anti-tumor drugs (MTDx4) for adriamycine and (MTDx2) for vincristine. The mice were given a single intraperitoneal injection with 40mg/kg body weight adriamycine or a single intraperitoneal injection of 20 mg/kg body weight vincristine at Day 0. Lot 1 in figure 28 shows the survival curve of the mice injected with either the MTDx2 of vincristine or either the MTDx4 of adriamycine.

Extract A according to the invention was injected intraperitoneally at a dose of 10 mg/kg body weight prior to the injection of the anti-tumor drugs. The following different schedules were applied.
- Extract A was injected on the same day (i.e. at Day 0), but 4 hours prior to the injection of the therapeutic compound adriamycine or vincristine. Results of this treatment are represented by lot 2, in Figure 28.
- Extract A was injected once a day during eight days prior to the day of injecting the therapeutic compound adriamycine or vincristine. Results of this treatment are represented by lot 3, in Figure 28.
- Extract A was injected once a day during eight days prior to the day of injecting the therapeutic compound adriamycine or vincristine and was further once a day during seven days after the injection of the anti-tumor drugs. Results of this treatment are represented by lot 4, in Figure 28.

As can be seen in Figure 28, all schedules wherein Extract A is used in combination with a therapeutic compound significantly prolong the survival of the mice as compared to injection of the therapeutic compounds as single agents.

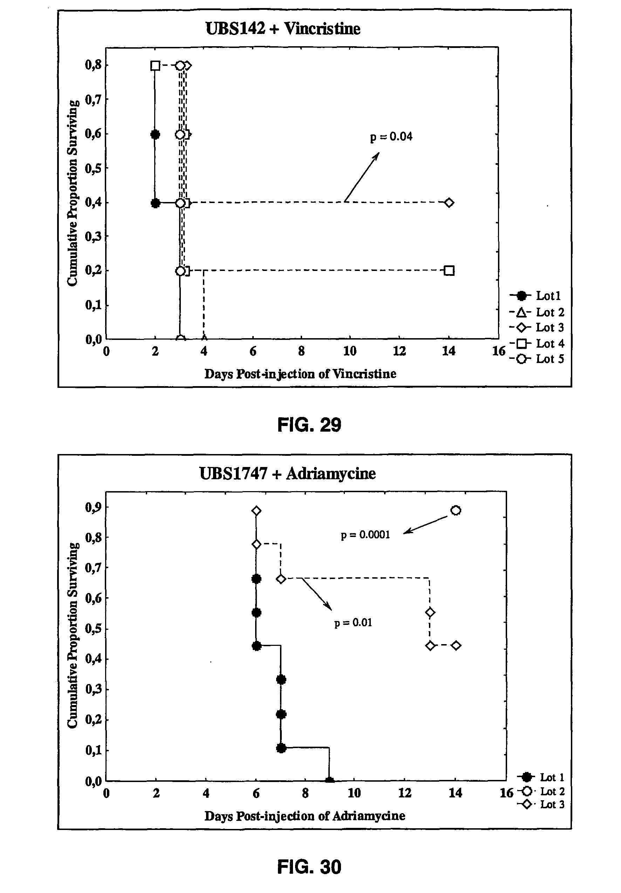

A second example illustrates the anti-poisonous effect of Extract A with another administration schedule. The mice were given a single intraperitoneal injection with 20 mg/kg body weight vincristine at Day 0. Lot 1 in Figure 29 shows the survival curve of the mice injected with of vincristine.

Extract A was injected intraperitoneally at a dose of 10 mg/kg body weight prior to or in the same time than the injection of anti-tumor drug. The following different schedules were applied.
- Extract A was injected on the same day (i.e. at Day 0), but 6 hours prior to the injection of the therapeutic compound vincristine. Results of this treatment are represented by lot 2, in Figure 29.
- Extract A was injected on the same day (i.e. at Day 0), but 4 hours prior to the injection of the therapeutic compound vincristine. Results of this treatment are represented by lot 3, in Figure 29.
- Extract A was injected on the same day (i.e. at Day 0), but 2 hours prior to the injection of the therapeutic compound vincristine. Results of this treatment are represented by lot 4, in Figure 29.
- Extract A was injected on the same day (i.e. at Day 0) and in the same time than the injection of the therapeutic compound vincristine. Results of this treatment are represented by lot 5, in Figure 29.

The parameter, which was applied for determining the protective, i.e. anti-poisonous effect of Extract A, on the toxic effects induced by the anti-tumor drugs consisted of the "prolongation of survival". Statistical analysis was performed by means of Kaplan-Meier statistics. The level of significance was p<0.05.

As can be seen in figure 29, the schedule wherein Extract A was injected 4 hours prior to the injection of vincistine (lot 3) significantly prolongs the survival of mice as compared to injection of the therapeutic compound as single agent.

It can thus be concluded that Extract A enables to significantly protect mice from the toxic effects of a mortal dose of frequently used anti-tumor compound such as adriamycine or vincristine. Extract A according to the invention has thus an anti-poisonous effect, by which enables to reduce the toxic effects of well-known anti-tumor compound.

A third example illustrates the anti-poisonous effect of Extract B Female mice of 4 to 5 weeks of age were injected of four well-known and clinically used anti-tumor compound: adriamycine, vincristine, oxaliplatine and camptothecine. The mice were given a single intraperitoneal injection with 20mg/kg body weight adriamycine or a single intraperitoneal injection of 20 mg/kg body weight vincristine or a single intraperitoneal injection of 40 mg/kg body weight oxaliplatine or a single intraperitoneal injection of 20 mg/kg body weight camptothecine at Day 0.
Lot 1 in Figure 30, Figure 31, Figure 32, Figure 33 shows the survival curve of the mice injected with either adriamycine or either vincristine or either oxaliplatine or either camptothecine.
Lot 2 in Figures 30 to 33 shows the "survival point" of the mice injected with Extract B at 10 mg/kg.
Lot 3 in Figures 30 to 33 show the survival curve of the mice injected intraperitoneally with Extract B at 10 mg/kg body weight on the same day (i.e. at Day 0) but 4 hours prior to the injection of the therapeutic compound.

As can be seen in figures 30 to 33, Extract B used in combination with a therapeutic compound significantly prolongs the survival of the mice as compared to injection of the therapeutic compound as single agents. Extract B didn't show cytotoxic properties. Indeed, no mouse died when it was injected with Extract B as single agent. Statistical analysis was performed by means of Kaplan-Meier statistics. The level of significance was p<0.05 for each combination Extract B and therapeutic compound.

It can thus be concluded that Extract B provides significant protection to mice from the toxic effects of a mortal dose of frequently used anti-tumor drugs. Extract B has thus an anti-poisonous effect, which enables the reduction of the toxic effects of well-known anti-tumor drugs. The anti-poisonous activity was preserved in Extract B as in Extract A.

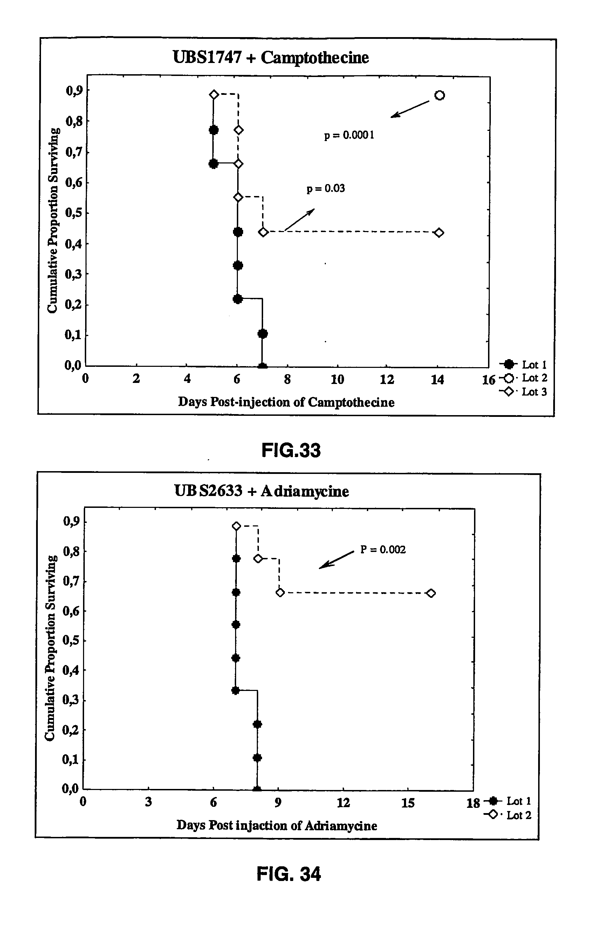

A fourth example illustrates the anti-poisonous effect of Extract C. Extract C was used in combination of adriamycine at 20 mg/kg body weight.
Lot1 in Figure 34 shows the survival curve of the mice injected with 20 mg/kg body weight of adriamycine.
Lot 2 in Figure 34 show the survival curve of the mice injected with Extract C at 10 mg/kg on the same day (i.e. at Day 0) but 4 hours prior to the injection of the therapeutic compound.

As can be seen in Figure 34, the injection of Extract C induced a significant survival prolongation of the mice as compared to the injection of the therapeutic compound as single agent. It can be thus concluded that Extract C show an anti-poisonous properties as the previous Extract B.

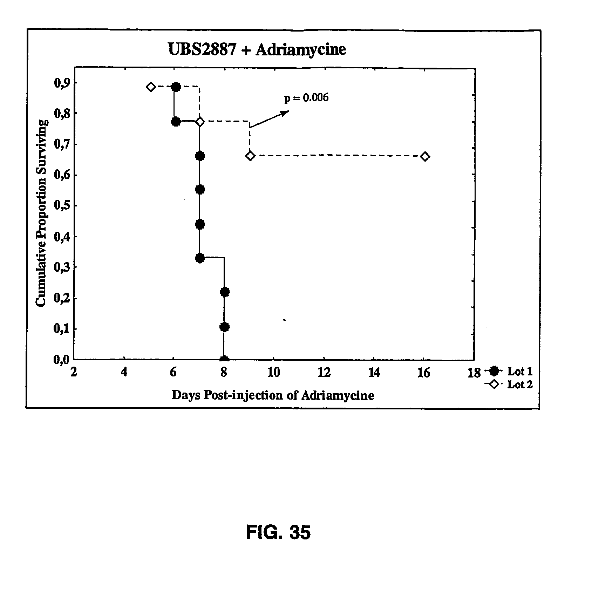

A fifth example illustrates the anti-poisonous effect of Extract D. Female mice of 4 to 5 weeks of age were injected with adriamycine. The mice were given a single intraperitoneal injection with 20 mg/kg body weight adriamycine at Day 0 (Lot 1 in Figure 35). Lot 2 in figure 35 shows the survival curve of the mice injected with Extract D intraperitoneally at a dose of 5 mg/kg body weight on the same day (i.e. at Day 0), but 4 hours prior to the injection of the therapeutic compound adriamycine.

As can be seen in Figure 35, the injection of Extract D induced a significant survival prolongation of the mice as compared to the injection of the therapeutic compound as single agent. It can be thus concluded that Extract D show an anti-poisonous properties as the previous Extract C.

In conclusion, these experiments illustrate that the purified extracts according to the invention prolong significantly the survival of mice as compared to the mice injected with therapeutic compound as single agent. Extract D, the most purified extract, showed an anti-poisonous properties on the toxic effects induced by the anti-tumor drugs.

## Claims

1. Use of a composition comprising an extract of *Calotropis procera,* and at least one therapeutic compound selected from the group comprising an anticancer-agent, a cytotoxic antibody or a fragment thereof, a cytotoxic hormone or a fragment thereof, and a cytotoxic peptide or a fragment thereof; for the preparation of a medicament for treating cancer.

2. Use of a composition according to claim 1, wherein said extract is obtained using an extraction procedure, comprising the steps of:
a) extracting the starting material of said *Calotropis procera* plant, said starting material being selected among fruits, aerial parts subterranean parts, and their mixtures, in an aliphatic alcohol, by dissolving the starting material in said alcohol thereby obtaining a suspension of said material in said alcohol, stirring said suspension, and filtering said suspension by fritted glass thereby obtaining a first filtrate and a first solid part;
b) extracting said first solid part in an aliphatic alcohol thereby obtaining a second filtrate and a second solid part;
c) combining said first and said second filtrate thereby obtaining a combined filtrate, and evaporating said combined filtrate under vacuum thereby obtaining said extract.

3. Use of a composition according to claim 1 or 2, where said extract comprises at least two active compounds selected from the group comprising asclepin, calactin, vorusharin, calotropin, calotropagenin, uzarigenin, calotoxin, usharin, usharidin, and 2"oxo-vorusharin.

4. Use of a composition according to any of claims 1 to 3, wherein the weight ratio of extract: therapeutic compound is in the range 0.001 : 1 to 1000 : 1.

5. Use of a composition according to any of claims 1 to 4, wherein said cancer is selected from the group comprising breast cancer, lymphoma, sarcoma, pancreatic cancer, melanoma, colorectal cancer, glioma, non small cell lung cancer, small cell lung cancer, skin cancer, bone cancer, ovarian cancer, CNS cancer, renal cancer, bladder cancer, head and neck cancer, prostate cancer, liver cancer, hematological cancers.

6. Use of a composition according to any of claims 1 to 5, wherein said therapeutic compound(s) is an anti-cancer agent.

7. Use of a composition according to any of claims 1 to 6, wherein said therapeutic compound is selected from the group comprising adriamycin, alkeran, ara-c, bleomycin, carmustine, busulfan, lomustine, camptothecine, carboplatinum, cisplatinum, cyclophosphamide, cytoxan, daunorubicin, dacarbazine, fluorouracil, fludarabine, gemcitabine (gemzar), herceptin, hexamethylmelamine, hydrea, idarubicin, ifosfamide, irinotecan (camptosar, CPT-11), leustatin, methotrexate, mithramycin, mitomycin, mitoxantrone, muphoran, navelbine, nitrogen mustard, oxaliplatine, rituxan, imatinib, streptozocine, taxol, taxotere, topotecan (hycamtin), velban, vincristine, etoposide, xeloda (capecitabine), or zevelin.

8. Use of a composition according to any of claims 1 to 6, wherein said therapeutic compound(s) is a cytotoxic antibody or a fragment thereof.

9. Use of a composition according to any of claims 1 to 6, wherein said therapeutic compound(s) is a cytotoxic hormone or a fragment thereof.

10. Use of a composition according to any of claims 1 to 6, wherein said therapeutic compound(s) is a cytotoxic peptide or a fragment thereof.

11. Use of a composition according to any of claims 1 to 10, wherein said extract is administered prior to, after, or at the same time as said therapeutic compound(s).

12. An extraction process for obtaining an extract having biologically active components comprising the steps of:
a) extracting the starting material of said *Calotropis procera* plant, said starting material being selected among fruits, aerial parts, subterranean parts, and their mixtures, in an aliphatic alcohol, by dissolving the starting material in said alcohol thereby obtaining a suspension of said material in said alcohol, stirring said suspension; and filtering said suspension by fritted glass thereby obtaining a first filtrate and a first solid part;
b) extracting said first solid part in an aliphatic alcohol thereby obtaining a second filtrate and a second solid part;
c) combining said first and said second filtrate thereby obtaining a combined filtrate; and
d) evaporating said combined filtrate under vacuum thereby obtaining said extract.

13. Active extract isolated from the process according to claim 12.

14. A kit comprising a container in which an extract of *Calotropis procera* as defined in any of claims 1 to 11 is present, and a container in which a therapeutic compound as defined in claim 1 is present.

## Patentansprüche

1. Verwendung einer Zusammensetzung umfassend einen Extrakt von *Calotropis procera* und mindestens einer therapeutischen Verbindung ausgewählt aus der Gruppe umfassend ein Antikrebsmittel, einen cytotoxischen Antikörper oder ein Fragment davon, ein cytotoxisches Hormon oder ein Fragment davon und ein cytotoxisches Peptid oder ein Fragment davon für die Herstellung eines Medikamentes zur Behandlung von Krebs.

2. Verwendung einer Zusammensetzung nach Anspruch 1, wobei der Extrakt durch ein Extraktionsverfahren erhalten wird, das die Schritte umfasst:
a) Extrahieren des Ausgangsmaterials von der *Calotropis procera-*Pflanze, wobei das Ausgangsmaterial ausgewählt ist aus Früchten, oberirdischen Teilen, unterirdischen Teilen und ihren Gemischen in einem aliphatischen Alkohol durch Auflösen des Ausgangsmaterials in dem Alkohol, wobei eine Suspension des Materials in Alkohol erhalten wird, Rühren der Suspension und Filtrieren der Suspension durch eine Glasfritte, wobei ein erstes Filtrat und ein erster fester Anteil erhalten werden;
b) Extrahieren des ersten festen Anteils in einem aliphatischen Alkohol, wobei ein zweites Filtrat und ein zweiter fester Anteil erhalten werden;
c) Vereinigen des ersten und zweiten Filtrats, wobei ein vereinigtes Filtrat erhalten wird und Eindampfen des vereinigten Filtrats unter Vakuum, wobei der Extrakt erhalten wird.

3. Verwendung einer Zusammensetzung nach Anspruch 1 oder 2, wobei der Extrakt mindestens zwei Wirkstoffe umfasst, die ausgewählt sind aus der Gruppe umfassend Asclepin, Calactin, Vorusharin, Calotropin, Calotropagenin, Uzarigenin, Calotoxin, Usharin, Usharidin, und 2"-Oxo-Vorusharin.

4. Verwendung einer Zusammensetzung nach einem der Ansprüche 1 bis 3, wobei das Gewichtsverhältnis von Extrakt : therapeutischer Verbindung im Bereich von 0,001 : 1 bis 1000 : 1 liegt.

5. Verwendung einer Zusammensetzung nach einem der Ansprüche 1 bis 4, wobei der Krebs ausgewählt ist aus der Gruppe umfassend Brustkrebs, Lymphom, Sarcom, Bauchspeicheldrüsenkrebs, Melanom, Dickdarmkrebs, Gliom, nicht-kleinzelligem Lungenkrebs, kleinzelligem Lungenkrebs, Hautkrebs, Knochenkrebs, Eierstockkrebs, Krebs des Zentralnervensystems, Nierenkrebs, Blasenkrebs, Kopf- und Nackenkrebs, Prostatakrebs, Leberkrebs, hämatologische Krebserkrankungen.

6. Verwendung einer Zusammensetzung nach einem der Ansprüche 1 bis 5, wobei die therapeutische Verbindung(en) ein Antikrebswirkstoff ist(sind).

7. Verwendung einer Zusammensetzung nach einem der Ansprüche 1 bis 6, wobei der Wirkstoff ausgewählt ist aus der Gruppe umfassend Adriamycin, Alkeran, Ara-c, Bleomycin, Carmustin, Busulfan, Lomustin, Camptothecin, Carboplatin, Cisplatin, Cyclophosphamid, Cytoxan, Daunorubicin, Dacarbazin, Fluoruracil, Fludarabin, Gemcitabin (Gemzar), Herceptin, Hexamethylmelamin, Hydrea, Idarubicin, Ifosfamid, Irinotecan (Camptosar, CPT-11), Leustatin, Methotrexat, Mithramycin, Mitomycin, Mitoxantron, Muphoran, Navelbin, Stickstoff-Lost, Oxaliplatin, Rituxan, Imatinib, Streptozocin, Taxol, Taxotere, Topotecan (Hycamtin), Velban, Vincristin, Etoposid, Xeloda (Capecitabin) oder Zevelin.

8. Verwendung einer Zusammensetzung nach einem der Ansprüche 1 bis 6, wobei die therapeutische Verbindung(en) ein cytotoxischer Antikörper oder ein Fragment davon ist(sind).

9. Verwendung einer Zusammensetzung nach einem der Ansprüche 1 bis 6, wobei die therapeutische Verbindung(en) ein cytotoxisches Hormon oder ein Fragment davon ist(sind).

10. Verwendung einer Zusammensetzung nach einem der Ansprüche 1 bis 6, wobei die therapeutische Verbindung(en) ein cytotoxisches Peptid oder ein Fragment davon ist(sind).

11. Verwendung einer Zusammensetzung nach einem der Ansprüche 1 bis 10, wobei der Extrakt vor, nach oder gleichzeitig mit der (den) therapeutischen Verbindung(en) verabreicht wird.

12. Extraktionsverfahren zum Erhalten eines Extrakts, der biologisch aktive Komponenten enthält, umfassend die Schritte:
a) Extrahieren des Ausgangsmaterials der *Calotropis* procera-Pflanze, wobei das Ausgangsmaterial ausgewählt wird aus Früchten, oberirdischen Teilen, unterirdischen Teilen und ihren Gemischen in einem aliphatischen Alkohol durch Auflösen des Ausgangsmaterials in dem Alkohol, wobei eine Suspension des Materials in Alkohol erhalten wird, Rühren der Suspension; und Filtrieren der Suspension durch eine Glasfritte, wobei ein erstes Filtrat und ein erster fester Anteil erhalten werden;
b) Extrahieren des ersten festen Anteils in einem aliphatischen Alkohol, wobei ein zweites Filtrat und ein zweiter fester Anteil erhalten werden;
c) Vereinen des ersten und zweiten Filtrats, wobei ein vereintes Filtrat erhalten wird; und
d) Eindampfen des vereinten Filtrats unter Vakuum, wobei der Extrakt erhalten wird.

13. Aktiver Extrakt erhalten durch das Verfahren nach Anspruch 12.

14. Kit umfassend einen Container, in welchem ein Extrakt von *Calotropis procera,* wie in einem der Ansprüche 1 bis 11 definiert, vorliegt, und einen Container, in welchem eine therapeutische Verbindung, wie in Anspruch 1 definiert, vorliegt.

## Revendications

1. Utilisation d'une composition comprenant un extrait de *Calotropis procera,* et au moins un composé thérapeutique choisi dans le groupe comprenant un agent anti-cancéreux, un anticorps cytotoxique ou un fragment d'anticorps cytotoxique, une hormone cytotoxique ou un fragment d'hormone cytotoxique, et un peptide cytotoxique ou un fragment de peptide cytotoxique ; pour la préparation d'un médicament pour traiter le cancer.

2. Utilisation d'une composition selon la revendication 1, dans laquelle ledit extrait est obtenu en utilisant une procédé d'extraction, comprenant les étapes suivantes :
a) extraire la matière première de ladite plante *Calotropis procera,* ladite matière première étant choisie parmi les fruits, les parties aériennes, les parties souterraines, et leurs mélanges, dans un alcool aliphatique, en dissolvant la matière première dans ledit alcool, pour obtenir ainsi une suspension de ladite matière dans ledit alcool, en agitant ladite suspension et en filtrant ladite suspension sur verre fritté, pour obtenir ainsi un premier filtrat et une première partie solide ;
b) extraite ladite première partie solide dans un alcool aliphatique pour obtenir ainsi un second filtrat et une seconde partie solide ;
c) combiner ledit premier et ledit second filtrat pour obtenir ainsi un filtrat combiné, et évaporer ledit filtrat combiné sous vide pour obtenir ainsi ledit extrait.

3. Utilisation d'une composition selon la revendication 1 ou la revendication 2, dans laquelle ledit extrait comprend au moins deux composés actifs choisis dans le groupe comprenant l'asclépine, la calactine, la vorusharine, la calotropine, la calotropagénine, l'uzarigénine, la calotoxine, l'usharine, l'usharidine et la 2''oxo-vorusharine.

4. Utilisation selon l'une quelconque des revendications 1 à 3, dans laquelle le ratio en poids extrait: composé thérapeutique est compris dans la plage de 0,001 : 1 à 1 000 : 1.

5. Utilisation d'une composition selon l'une quelconque des revendications 1 à 4, dans laquelle ledit cancer est choisi dans le groupe comprenant le cancer du sein, le lymphome, le sarcome, le cancer du pancréas, le mélanome, le cancer colorectal, le gliome, le cancer du poumon non à petites cellules, le cancer du poumon à petites cellules, le cancer de la peau, le cancer des os, le cancer de l'ovaire, un cancer du SNC, le cancer du rein, le cancer de la vessie, un cancer de la tête et du cou, le cancer de la prostate, le cancer du foie, les cancers hématologiques.

6. Utilisation d'une composition selon l'une quelconque des revendications 1 à 5, dans laquelle ledit ou lesdits composés thérapeutiques sont des agents anti-cancéreux.

7. Utilisation selon l'une quelconque des revendications 1 à 6, dans laquelle ledit composé thérapeutique est choisi dans le groupe comprenant l'adriamycine, alkéran, l'ara-c, la bléomycine, la carmustine, le busulfan, la lomustine, la camptothécine, le carboplatine, le cisplatine, le cyclophosphamide, cytoxan, la daunorubicine, la dacarbazine, le fluorouracil, la fludarabine, le gemcitabine (gemzar), l'herceptine, l'hexaméthylmélanine, hydréa, l'idarubicin, l'ifosfamide, l'irinotécan (camptosar, CPT-11), leustatine, le méthotrexate, la mithramycine, la mitomycine, la mitoxantrone, muphoran, navelbine, la moutarde azotée, l'oxaliplatine, rituxan, l'imatinib, la streptozocine, le taxol, taxotère, le topotécan (hycamtine), velban, la vincristine, l'étoposide, xeloda (capécitabine) ou zevelin.

8. Utilisation d'une composition selon l'une quelconque des revendications 1 à 6, dans laquelle ledit ou lesdits composés thérapeutiques sont un anticorps cytotoxique ou un fragment d'anticorps cytotoxique.

9. Utilisation d'une composition selon l'une quelconque des revendications 1 à 6, dans laquelle ledit ou lesdits composés thérapeutiques sont une hormone cytotoxique ou un fragment d'hormone cytotoxique.

10. Utilisation d'une composition selon l'une quelconque des revendications 1 à 6, dans laquelle ledit ou lesdits composés thérapeutiques sont un peptide cytotoxique ou un fragment de peptide cytotoxique.

11. Utilisation d'une composition selon l'une quelconque des revendications 1 à 10, dans laquelle ledit extrait est administré avant, après ou en même temps que ledit ou lesdits composés thérapeutiques.

12. Procédé d'extraction pour obtenir un extrait ayant des composants actifs sur le plan biologique comprenant les étapes suivantes :
a) extraire la matière première de ladite plante *Calotropis procera,* ladite matière première étant choisie parmi les fruits, les parties aériennes, les parties souterraines et leurs mélanges, dans un alcool aliphatique, en dissolvant la matière première dans ledit alcool pour obtenir ainsi une suspension de ladite matière dans ledit alcool, en agitant ladite suspension ; et en filtrant ladite suspension sur verre fritté, pour obtenir ainsi un premier filtrat et une première partie solide ;
b) extraire ladite première partie solide dans un alcool aliphatique pour obtenir ainsi un second filtrat et une seconde partie solide ;
c) combiner ledit premier et ledit second filtrat pour obtenir ainsi un filtrat combiné ; et
d) évaporer ledit filtrat combiné sous vide pour obtenir ainsi ledit extrait.

13. Extrait actif isolé par le procédé selon la revendication 12.

14. Trousse comprenant un récipient dans lequel un extrait de *Calotropis procera* comme défini dans l'une quelconque des revendications 1 à 11 est présent, et un récipient dans lequel un composé thérapeutique comme défini dans la revendication 1 est présent.
